# EUROPEAN PATENT APPLICATION

(11) **EP 4 215 549 A1**
(43) Date of publication of application: **26.07.2023**
(21) Application number: 21868693.9
(22) Date of filing: 17.09.2021
(51) Int. Cl.: C07K 16/46, C12N 15/13, A61K 39/395, A61K 47/68

(54) **ANTI-4-1BB-ANTI-PD-L1 BISPECIFIC ANTIBODY, AND PHARMACEUTICAL COMPOSITION AND USE THEREOF**

(30) Priority: 17.09.2020 CN 202010983606
(71) Applicant: Biotheus Inc., Tangjiawan Town, Xiangzhou District Zhuhai, Guangdong 519080 (CN)
(72) Inventor: ZHAI, Tianhang, ZHUHAI, Guangdong 519080 (CN); MIAO, Xiaoniu, ZHUHAI, Guangdong 519080 (CN); XU, Yingda, ZHUHAI, Guangdong 519080 (CN); WANG, Tao, ZHUHAI, Guangdong 519080 (CN); TSUN, Andy, ZHUHAI, Guangdong 519080 (CN); HUANG, Weifeng, ZHUHAI, Guangdong 519080 (CN)
(74) Representative: Cabinet Nony
(86) International application number: PCT/CN2021/118908
(87) International publication number: WO 2022/057871

(57) **Abstract**

The present invention relates to an anti-4-1BB-anti-PD-L1 bispecific antibody, and a pharmaceutical composition and the use thereof. Specifically, the present invention relates to a bispecific antibody, which comprises a first protein functional region targeting 4-1BB and a second protein functional region targeting PD-L1 or PD-1, wherein the first protein functional region is a single-domain antibody against 4-1BB; and the second protein functional region is an anti-PD-L1 antibody, anti-PD-1 antibody, anti-CTLA-4 antibody or anti-HER-2 antibody, or an antigen-binding fragment thereof. The bispecific antibody has the function of blocking the binding of PD-L1 to a receptor PD-1, and binds to 4-1BB on an immune cell, such that the immune cell activity in a tumor microenvironment is activated and the effect of inhibiting tumor occurrence and development is improved more effectively.

## Description

### Technical Field

The present invention belongs to the field of biomedicine, and relates to an anti-4-1BB-anti-PD-L1 bispecific antibody, and pharmaceutical composition and use thereof.

### Background Art

Tumor necrosis factor receptor superfamily member 4-1BB, also known as CD137 or TNFRF9, is a member of the TNF receptor family. 4-1BB is a type I transmembrane protein with 255 amino acids (NCBI: NP_001552), which consists of an N-terminal signal peptide containing 17 amino acids, an extracellular region of 169 amino acids, a transmembrane region of 27 amino acids, and a C-terminal intracellular region of 42 amino acids. 4-1BB is mainly expressed in activated T cells, NK cells, regulatory T cells, dendritic cells, monocytes, neutrophils and eosinophils, and endothelial cells of tumor vessels have also been reported to express 4-1BB. Therefore, in the treatment of tumors and some autoimmune diseases, 4-1BB is also a potential target. Specifically, in preclinical animal models such as colorectal cancer, lung cancer, breast cancer, and melanoma, agonist molecule targeting 4-1BB shows significant anti-tumor activity as a single drug or in combination with an additional antibody such as anti-PD-1, anti-PD-L1, anti-CTLA-4, anti-HER-2 (Etxeberna I, et al. ESMO Open 2020;4:e000733.).

Programmed death 1 ligand 1 (PD-L1), also known as CD274, is a member of the B7 family and a ligand of PD-1. PD-L1 is a type I transmembrane protein with a total of 290 amino acids, comprising an IgV-like region, an IgC-like region, a transmembrane hydrophobic region and an intracellular region consisting of 30 amino acids. Unlike other B7 family molecules, PD-L1 negatively regulates the immune response. Studies have found that PD-L1 is mainly expressed in activated T cells, B cells, macrophages, and dendritic cells. In addition to lymphocytes, PD-L1 is also expressed in other endothelial cells of many tissues such as thymus, heart, placenta, etc., and various non-lymphoid systems such as melanoma, liver cancer, gastric cancer, renal cell carcinoma, ovarian cancer, colon cancer, breast cancer, esophageal cancer, head and neck cancer, etc. (Akintunde Akinleye & Zoaib Rasool, Journal of Hematology & Oncology volume 12, Article number: 92 (2019)). PD-L1 has a certain generality in regulating autoreactive T and B cells and immune tolerance, and plays a role in peripheral tissue T and B cell responses. High expression of PD-L1 on tumor cells is associated with poor prognosis of cancer patients.

Most of the antibody drugs currently on the market are monoclonal antibodies. Therapeutic monoclonal antibodies have been used to treat cancer, autoimmune diseases, inflammation and other diseases, and most of them are specific to one target. However, patients may become resistant or unresponsive to monoclonal antibody therapy. And some diseases are affected by multiple factors in the body, including different signaling pathways, different regulatory mechanisms of cytokines and receptors, etc., and single-target immunotherapy does not seem to be enough to destroy cancer cells. Therefore, it needs to be achieved by combining different drugs or multiple targeting strategies using multispecific antibodies.

Although bifunctional antibodies are a direction in the development of antibody drugs, they face many challenges, such as preclinical evaluation models, low expression, poor stability, complex processes, and large differences in quality control. Therefore, the development of bifunctional antibodies has always been difficult.

Therefore, it is necessary to develop a bispecific antibody targeting PD-L1 and 4-1BB with good specificity, good curative effect and easy preparation.

### Contents of the present invention

After in-depth research and creative work, the inventors have obtained an anti-4-1BB-anti-PD-L1 bispecific antibody (hereinafter also expressed as anti-PD-L1/4-1BB bispecific antibody or anti-4-1BB/PD-L1 bispecific antibody). The inventors surprisingly found that the bispecific antibody of the present invention has the function of blocking the binding of PD-L1 to the receptor PD-1; at the same time, the bispecific antibody can also bind to 4-1BB on immune cells, activate immune cells in the tumor microenvironment, thereby more effectively improve the effect of inhibiting tumor occurrence and development, and thus has excellent anti-tumor activity and good safety. The following invention is thus provided:

One aspect of the present invention relates to a bispecific antibody, which comprises:
a first protein functional region targeting 4-1BB, and
a second protein functional region targeting PD-L1 or PD-1;
wherein:
   the first protein functional region is an anti-4-1BB single-domain antibody;
the second protein functional region is an anti-PD-L1 antibody, an anti-PD-1 antibody, an anti-CTLA-4 antibody or an anti-HER-2 antibody, or an antigen-binding fragment thereof.

In some embodiments of the present invention, in the bispecific antibody, the anti-4-1BB single-domain antibody comprises a heavy chain variable region, and the heavy chain variable region comprises CDR1 with an amino acid sequence as set forth in SEQ ID NO: 16, CDR2 with an amino acid sequence as set forth in SEQ ID NO: 17, and CDR3 with an amino acid sequence as set forth in SEQ ID NO: 18;
preferably, the anti-4-1BB single-domain antibody has an amino acid sequence as set forth in SEQ ID NO: 2.

In some embodiments of the present invention, in the bispecific antibody, the second protein functional region is an anti-PD-L1 monoclonal antibody, an anti-PD-1 monoclonal antibody, an anti-CTLA-4 monoclonal antibody or an anti-HER-2 monoclonal antibody.

In some embodiments of the present invention, in the bispecific antibody, the second protein functional region is an anti-PD-L1 single-chain antibody, an anti-PD-1 single-chain antibody, an anti-CTLA-4 single-chain antibody or an anti-HER-2 single-chain antibody.

In some embodiments of the present invention, in the bispecific antibody, the second protein functional region is an anti-PD-L1 single-domain antibody, an anti-PD-1 single-domain antibody, an anti-CTLA-4 single-domain antibody or an anti-HER-2 single-domain antibody;
preferably, the anti-PD-L1 single-domain antibody comprises a heavy chain variable region, the heavy chain variable region comprises CDR1 with an amino acid sequence as set forth in SEQ ID NO: 19, CDR2 with an amino acid sequence as set forth in SEQ ID NO: 20, and CDR3 with an amino acid sequence as set forth in SEQ ID NO: 21;
preferably, the anti-PD-L1 single-domain antibody has an amino acid sequence as set forth in SEQ ID NO: 4.

In the present invention, the CDRs of the anti-4-1BB single-domain antibody and anti-PD-L1 single-domain antibody are defined by the IMGT numbering system, please refer to Ehrenmann F, Kaas Q, Lefranc M P. IMGT/3Dstructure-DB and IMGT/DomainGapAlign: a database and a tool for immunoglobulins or antibodies, T cell receptors, MHC, IgSF and MhcSF [J]. Nucleic acids research,2009; 38 (suppl_1): D301-D307.

In some embodiments of the present invention, in the bispecific antibody,
the anti-4-1BB single-domain antibody comprises a heavy chain variable region, the heavy chain variable region comprises CDR1 with an amino acid sequence as set forth in SEQ ID NO: 16, CDR2 with an amino acid sequence as set forth in SEQ ID NO: 17, and CDR3 with an amino acid sequence as set forth in SEQ ID NO: 18, and
the anti-PD-L1 single-domain antibody comprises a heavy chain variable region, the heavy chain variable region comprises CDR1 with an amino acid sequence as set forth in SEQ ID NO: 19, CDR2 with an amino acid sequence as set forth in SEQ ID NO: 20, and CDR3 with an amino acid sequence as set forth in SEQ ID NO: 21.

In some embodiments of the present invention, in the bispecific antibody,
the anti-4-1BB single-domain antibody has an amino acid sequence as set forth in SEQ ID NO: 2, and
the anti-PD-L1 single-domain antibody has an amino acid sequence as set forth in SEQ ID NO: 4.

In some embodiments of the present invention, in the bispecific antibody, the bispecific antibody further comprises one or more (e.g., 2 or 3) Fc fragments of IgG, such as Fc fragment of IgG1, IgG2, IgG3 or IgG4;
or, the bispecific antibody further comprises one or more (e.g., 2 or 3) constant regions of IgG, such as heavy chain constant region of IgG1, IgG2, IgG3 or IgG4;
preferably, the Fc fragment of IgG or constant region of IgG is located between the first protein functional region and the second protein functional region.

In some embodiments of the present invention, the bispecific antibody consists of the first protein functional region, the second protein functional region, the Fc fragment of IgG, and an optional linker.

In some embodiments of the present invention, the bispecific antibody consists of the first protein functional region, the second protein functional region, the Fc fragment of IgG1, and an optional linker.

In some embodiments of the present invention, the bispecific antibody comprises one Fc fragment of IgG1, and the Fc fragment of IgG1 is located between the first protein functional region and the second protein functional region.

In some embodiments of the present invention, in the bispecific antibody, the Fc fragment of IgG or the heavy chain constant region of IgG is ligated to the C-terminal of the first protein functional region, and/or is ligated to the C-terminal of the second protein functional region;
the Fc fragment of IgG or the heavy chain constant region of IgG is ligated directly or through a linker to the first protein functional region; and the Fc fragment of IgG or the heavy chain constant region of IgG is ligated directly or through a linker to the second protein functional region;
preferably, the linker has an amino acid sequence independently selected from SEQ ID NO: 5 and SEQ ID NO: 22.

In some embodiments of the present invention, in the bispecific antibody, the Fc fragment of IgG1 is ligated to the C-terminal of the first protein functional region, and/or ligated to the C-terminal of the second protein functional region;
the Fc fragment of IgG1 is ligated directly or through a linker to the first protein functional region; and the Fc fragment of IgG1 is ligated directly or through a linker to the second protein functional region;
preferably, the amino acid sequence of the connecting fragment is independently selected from SEQ ID NO: 5 and SEQ ID NO: 22.

In some embodiments of the present invention, in the bispecific antibody, according to the EU numbering system, the Fc fragment of IgG or the heavy chain constant region of IgG comprises a L234A mutation and a L235A mutation; optionally, the Fc fragment of IgG also comprises a G237A mutation;
preferably, the Fc fragment of IgG is a Fc fragment of IgG1 comprising a L234A mutation and a L235A mutation; optionally, the Fc fragment of IgG1 further comprises a G237A mutation; preferably, the Fc fragment of IgG1 has an amino acid sequence as set forth in SEQ ID NO: 3;
preferably, the Fc fragment of IgG further comprises a Knob-in-hole mutation;
preferably, the Fc fragment of IgG1 further comprises a Knob-in-hole mutation; preferably, the Fc fragment of IgG1 has an amino acid sequence as set forth in SEQ ID NO: 8 or SEQ ID NO: 9.

In some embodiments of the present invention, in the bispecific antibody, the bispecific antibody comprises the following first peptide chain:
the first peptide chain that comprises the first protein functional region, the Fc fragment of IgG or the heavy chain constant region of IgG, an optional linker, and the second protein functional region sequentially from the N-terminal to the C-terminal; or comprises the second protein functional region; the Fc fragment of IgG or the heavy chain constant region of IgG, an optional linker, and the first protein functional region. Preferably, the linker has an amino acid sequence independently selected from SEQ ID NO: 5 and SEQ ID NO: 22.

In some embodiments of the present invention, in the bispecific antibody, the bispecific antibody comprises the following first peptide chain:
the first peptide chain that comprises the first protein functional region, the Fc fragment of IgG1 or the heavy chain constant region of IgG1, an optional linker, and the second protein functional region sequentially from the N-terminal to the C-terminal; or comprises the second protein functional region, the Fc fragment of IgG1 or the heavy chain constant region of IgG1, an optional linker, and the first protein functional region. Preferably, the linker has an amino acid sequence independently selected from SEQ ID NO: 5 and SEQ ID NO: 22.

In some embodiments of the present invention, in the bispecific antibody, the Fc fragment of IgG1 is an Fc fragment of IgG1 comprising a L234A mutation and a L235A mutation (referred to as "LaLa mutation" for short); optionally, the Fc fragment of IgG1 further comprises a G237A mutation; preferably, the Fc fragment of IgG1 has an amino acid sequence as set forth in SEQ ID NO: 3.

In some embodiments of the present invention, in the bispecific antibody, the Fc fragment of IgG1 comprises a Knob-in-hole mutation; preferably, according to the EU numbering system, "Knob" refers to that the Fc undergoes S354C and T366W mutations; "hole" refers to that the Fc undergoes Y349C, T366S, L368A and Y407V mutations.

In some embodiments of the present invention, in the bispecific antibody, the Fc fragment of IgG1 comprises a L234A mutation, a L235A mutation and a Knob-in-hole mutation; preferably, the Fc fragment of IgG1 has an amino acid as set forth in SEQ ID NO: 8 or SEQ ID NO: 9.

In some embodiments of the present invention, in the bispecific antibody, the first peptide chain has an amino acid sequence as set forth in SEQ ID NO: 1 or SEQ ID NO: 6.

In some embodiments of the present invention, wherein the bispecific antibody is a dimer formed with two first peptide chains, preferably a dimer formed with the first peptide chains as set forth in SEQ ID NO: 1 and/or SEQ ID NO: 6;
preferably, the two first peptide chains are linked by 2 or 3 pairs of disulfide bonds;
optionally, the two first peptide chains also comprise a Knob-in-hole mutation.

In some embodiments of the present invention, in the bispecific antibody, one of the two first peptide chains comprise a Knob mutation, and the other comprises a hole mutation.

In some embodiments of the present invention, the bispecific antibody is a dimer formed with two first peptide chains a set forth in SEQ ID NO: 1.

In some embodiments of the present invention, the bispecific antibody is a dimer formed with two first peptide chains as set forth in SEQ ID NO: 6.

In some embodiments of the present invention, wherein, the bispecific antibody is a dimer formed with one first peptide chain set forth in SEQ ID NO: 1 and one first peptide chain set forth in SEQ ID NO: 6.

In some embodiments of the present invention, the bispecific antibody further comprises the following second peptide chain:
the second peptide chain that comprises the Fc fragment of IgG or the heavy chain constant region of IgG; preferably, the N-terminal and/or C-terminal of the second peptide chain are ligated directly or through a linker to the first protein functional region and/or the second protein functional region.

In some embodiments of the present invention, the bispecific antibody further comprises the following second peptide chain:
the second peptide chain that comprises the Fc fragment of IgG1 or the heavy chain constant region of IgG1; preferably, the N-terminal and/or C-terminal of the second peptide chain are ligated directly or through a linker to the first protein functional region and/or the second protein functional region.

In some embodiments of the present invention, in the bispecific antibody, the second peptide chain has an amino acid sequence as set forth in SEQ ID NO: 9 or SEQ ID NO: 10.

In some embodiments of the present invention, in the bispecific antibody, the first peptide chain and the second peptide chain form a dimer;
preferably, the first peptide chain and the second peptide chain are linked by 2 or 3 pairs of disulfide bonds;
preferably, the first peptide chain and the second peptide chain further comprise a Knob-in-hole mutation;
preferably, the first peptide chain has an amino acid sequence as set forth in SEQ ID NO: 7, and the second peptide chain has an amino acid sequence as set forth in SEQ ID NO: 9 or SEQ ID NO: 10.

In some embodiments of the present invention, in the bispecific antibody, the first peptide chain comprises a Knob mutation, and the second peptide chain comprises a hole mutation.

In some embodiments of the present invention, in the bispecific antibody, the first peptide chain comprises a hole mutation, and the second peptide chain comprises a Knob mutation.

Another aspect of the present invention relates to an isolated nucleic acid molecule, which encodes the bispecific antibody according to any one of the items of the present invention.

The present invention further relates to a vector, which comprises the isolated nucleic acid molecule according to the present invention.

The present invention also relates to a host cell, which comprises the isolated nucleic acid molecule according to the present invention, or the vector according to the present invention.

Another aspect of the present invention relates to a method for preparing the bispecific antibody according to any one of the items of the present invention, which comprises steps of: culturing the host cell according to the present invention under suitable conditions, and recovering the bispecific antibody from the cell culture.

Another aspect of the present invention relates to a conjugate, which comprises a bispecific antibody and a coupling moiety, wherein the bispecific antibody is the bispecific antibody according to any one of the items of the present invention, and the coupling moiety is a detectable label; preferably, the coupling moiety is a radioactive isotope, a fluorescent substance, a luminescent substance, a colored substance or an enzyme.

Another aspect of the present invention relates to a kit, which comprises the bispecific antibody according to any one of the items of the present invention, or comprises the conjugate according to the present invention;
preferably, the kit further comprises a second antibody capable of specifically binding to the bispecific antibody; optionally, the second antibody further comprises a detectable label, such as a radioactive isotope, a fluorescent substance, a luminescent substance, a colored substance or an enzyme.

Another aspect of the present invention relates to a use of the bispecific antibody according to any one of the items of the present invention in the manufacture of a kit for detecting the presence or level of 4-1BB and/or PD-L1 in a sample.

Another aspect of the present invention relates to a pharmaceutical composition, which comprises the bispecific antibody according to any one of the items of the present invention or the conjugate according to the present invention; optionally, the pharmaceutical composition further comprises a pharmaceutically acceptable excipient.

Another aspect of the present invention relates to a use of the bispecific antibody according to any one of the items of the present invention or the conjugate according to the present invention in the manufacture of a medicament for the prevention and/or treatment of a malignant tumor; preferably, the malignant tumor is selected from a group consisting of rectal cancer, colon cancer, lung cancer, melanoma, liver cancer, gastric cancer, renal cell carcinoma, ovarian cancer, esophageal cancer and head and neck cancer.

Another aspect of the present invention relates to a method for treating and/or preventing a malignant tumor, comprising a step of administering an effective amount of the bispecific antibody according to any one of the items of the present invention or the conjugate according to the present invention to a subject in need thereof; preferably, the malignant tumor is selected from a group consisting of rectal cancer, colon cancer,

In some embodiments of the present invention, in the method, the step of administering an effective amount of the bispecific antibody according to any one of the items of the present invention to the subject in need thereof is performed before or after surgical treatment, and/or before or after radiation therapy.

In some embodiments of the present invention, in the method,
the bispecific antibody of the present invention is administrated in a single dose of 0.1-100 mg, preferably 4.8-24 mg or 1-10 mg per kilogram body weight; or, the bispecific antibody of the present invention is administrated in a single dose of 10-1000mg, preferably 50-500mg, 100-400mg, 150-300mg, 150-250mg or 200mg;
preferably, the administration is performed once every 3 days, 4 days, 5 days, 6 days, 10 days, 1 week, 2 weeks or 3 weeks;
preferably, the administration is performed by intravenous drip or intravenous injection.

The bispecific antibody or the conjugate according to any one of the items of the present invention, which is used for treating and/or preventing a malignant tumor; preferably, the malignant tumor is selected from a group consisting of rectal cancer, colon cancer, lung cancer, melanoma, liver, stomach, renal cell, ovarian, esophageal, and head and neck cancers.

As used herein, the term "antibody" refers to an immunoglobulin molecule generally composed of two pairs of polypeptide chains, each pair having one "light" (L) chain and one "heavy" (H) chain. Antibody light chains can be classified as κ and λ light chains. Heavy chains can be classified as µ, δ, γ, α, or ε, and the isotypes of antibody are defined as IgM, IgD, IgG, IgA, and IgE, respectively. Within the light chain and heavy chain, variable and constant regions are linked by a "J" region of about 12 or more amino acids, and the heavy chain further comprising a "D" region of about 3 or more amino acids. Each heavy chain is composed of a heavy chain variable region (VH) and a heavy chain constant region (CH). The heavy chain constant region consists of 3 domains (CH1, CH2 and CH3). Each light chain is composed of a light chain variable region (VL) and a light chain constant region (CL). The light chain constant region consists of one domain, CL. The antibody constant regions mediate the binding of immunoglobulin to a host tissue or factor, including various cells (e.g., effector cells) of the immune system and first component (Clq) of the classical complement system. VH and VL regions can also be subdivided into regions with high variability (which are called as complementarity determining regions (CDRs)), among which more conserved regions called framework regions (FRs) are interspersed. Each VH and VL consists of 3 CDRs and 4 FRs arranged in the following order: FR1, CDR1, FR2, CDR2, FR3, CDR3, and FR4, from amino-terminal to carboxy-terminal. The variable regions (VH and VL) of each heavy chain/light chain pair form an antibody binding site, respectively. Assignment of amino acids to the regions or domains follows the definition of Bethesda M.d., Kabat Sequences of Proteins of Immunological Interest (National Institutes of Health, (1987 and 1991)), or Chothia & Lesk J. Mol. Biol. 1987;196:901-917; Chothia et al. Nature 1989;342:878-883, or the IMGT numbering system, see the definitions of Ehrenmann F, Kaas Q, Lefranc M P. IMGT/3Dstructure-DB and IMGT/DomainGapAlign: a database and a tool for immunoglobulins or antibodies, T cell receptors, MHC, IgSF and MhcSF[J]. Nucleic acids research, 2009;38(suppl_1):D301-D307.

The term "antibody" is not limited to any particular antibody production method. For example, it includes recombinant antibody, monoclonal antibody and polyclonal antibody. The antibody can be an antibody of different isotype, for example, IgG (e.g., IgG1, IgG2, IgG3, or IgG4 subtype), IgA1, IgA2, IgD, IgE, or IgM antibody.

As used herein, the terms "mAb" and "monoclonal antibody" refer to an antibody or a fragment of antibody derived from a group of highly homologous antibody molecules, that is, except for natural mutations that may occur spontaneously, a group of identical antibody molecules. The mAb is highly specific for a single epitope on an antigen. Compared with monoclonal antibody, a polyclonal antibody usually contains at least two or more different antibodies, and these different antibodies usually recognize different epitopes on an antigen. Monoclonal antibodies can usually be obtained using hybridoma technology that was first reported by Kohler et al. (Köhler G, Milstein C. Continuous cultures of fused cells secreting antibody of predefined specificity [J]. nature, 1975; 256(5517):495), and can also be obtained using recombinant DNA techniques (see, for example, U.S. Patent 4,816,567).

As used herein, the term "humanized antibody" refers to an antibody or an antibody fragment obtained by replacing all or part of the CDR regions of a human immunoglobulin (recipient antibody) with the CDR regions of a non-human antibody (donor antibody), wherein the donor antibody can be a non-human (e.g., mouse, rat, or rabbit) antibody with the desired specificity, affinity, or reactivity. In addition, some amino acid residues in the framework region (FR) of the recipient antibody can also be replaced by the corresponding amino acid residues of a non-human antibody, or by the amino acid residues of other antibodies, so as to further improve or optimize the performance of antibody. For more details on humanized antibodies, see, for example, Jones et al., Nature 1986; 321:522 525; Reichmann et al., Nature, 1988; 332:323329; Presta, Curr. Op. Struct. Biol. 1992; 2:593-596; and Clark, Immunol. Today 2000; 21: 397-402. In some cases, the antigen-binding fragment of antibody is a diabody, in which the V_{H} and V_{L} domains are expressed on a single polypeptide chain, but the linker used is too short to allow the pairing between two domains of the same chain, thereby forcing the domain to pair with a complementary domain of another chain and creating two antigen-binding sites (see, for example, Holliger P. et al., Proc. Natl. Acad. Sci. USA 1993; 90:6444 6448 and Poljak R. J. et al., Structure 1994; 2:1121 1123).

The fusion protein as described herein is a protein product co-expressed by two genes through DNA recombination. Methods for producing and purifying antibodies and antigen-binding fragments are well known in the art (e.g., Cold Spring Harbor's Antibody Laboratory Technique Guide, Chapters 5-8 and Chapter 15).

As used herein, the term "isolation" or "isolated" means acquisition from a natural state by an artificial means. If an "isolated" substance or component occurs in nature, the natural environment in which it exists has been altered, or the substance has been isolated from the natural environment, or both have occurred. For example, for an unisolated polynucleotide or polypeptide naturally existing in a living animal, the same polynucleotide or polypeptide with high purity isolated from this natural state is called isolated. The term "isolation" or "isolated" does not exclude the admixture of an artificial or synthetic substance, nor the presence of other impurities which do not affect the substance activity.

As used herein, the term "vector" refers to a nucleic acid delivery vehicle into which a polynucleotide can be inserted. When the vector is capable of achieving the expression of a protein encoded by the inserted polynucleotide, the vector is called an expression vector. A vector can be introduced into a host cell by transformation, transduction or transfection, so that a genetic material element it carries can be expressed in the host cell. Vectors are well known to those skilled in the art, including but not limited to: plasmid; phagemid; cosmid; artificial chromosome, such as yeast artificial chromosome (YAC), bacterial artificial chromosome (BAC) or P1-derived artificial chromosome (PAC); phage such as λ phage or M13 phage, and animal viruses. The animal viruses that can be used as vectors include, but are not limited to, retrovirus (including lentivirus), adenovirus, adeno-associated virus, herpesvirus (e.g., herpes simplex virus), poxvirus, baculovirus, papillomavirus, papovavirus (e.g., SV40). A vector may contain a variety of elements that control expression, including but not limited to, promoter sequence, transcription initiation sequence, enhancer sequence, selection element, and reporter gene. In addition, the vector may also contain a replication origin.

As used herein, the term "host cell" refers to a cell that can be used to introduce a vector, including but not limited to, prokaryotic cell such as *Escherichia coli* or *Bacillus subtilis,* fungal cell such as yeast cell or *Aspergillus*, insect cell such as *S2 Drosophila* cell or Sf9, or animal cell such as fibroblast, CHO cell, GS cell, COS cell, NSO cell, HeLa cell, BHK cell, HEK293 cell or human cell.

As used herein, the term "pharmaceutically acceptable excipient" refers to a carrier and/or excipient that is pharmacologically and/or physiologically compatible with the subject and the active ingredient, which are well known in the art (see, for example, Remington's Pharmaceutical Sciences. Edited by Gennaro AR, 19th ed. Pennsylvania: Mack Publishing Company, 1995), and include but are not limited to: pH adjuster, surfactant, adjuvant, ionic strength enhancer. For example, the pH regulator includes but is not limited to phosphate buffer; the surfactant includes but is not limited to cationic, anionic or nonionic surfactant, such as Tween-80; the ionic strength enhancer includes but is not limited to sodium chloride.

As used herein, the term "effective amount" refers to an amount sufficient to achieve, or at least partially achieve, the desired effect. For example, an effective amount for preventing a disease (e.g., a tumor) refers to an amount sufficient to prevent, arrest, or delay the occurrence of a disease (e.g., a tumor); an effective amount for treating a disease refers to an amount sufficient to cure or at least partially prevent a disease and complication thereof in a patient with the disease. Determining such an effective amount is well within the capability of those skilled in the art. For example, an effective amount for therapeutic use will depend on the severity of the disease to be treated, the general state of the patient's own immune system, the general condition of the patient such as age, weight and sex, the mode of administration of drug, other therapies administered concomitantly, and so on.

In the present invention, if there is no special description, the "first" (e.g., the first protein functional region) and "second" (e.g., the second protein functional region) are used for the purpose of distinction in reference or clarity of expression, and do not have a typical sequential implication.

The present invention also relates to any one of the following items 1 to 10:
1. A bispecific antibody, characterized in that the bispecific antibody comprises:
   (a) an anti-PD-L1 single-domain antibody; and
   (b) an anti-4-1BB single-domain antibody.
2. An isolated polynucleotide, characterized in that, the polynucleotide encodes the bispecific antibody according to item 1.
3. A vector, characterized in that, the vector comprises the polynucleotide according to item 2.
4. A host cell, characterized in that, the host cell comprises the vector according to item 3, or the polynucleotide according to item 2 is integrated into the genome of the host cell;
   or, the host cell expresses the bispecific antibody according to item 1.
5. A method for producing the bispecific antibody according to item 1, comprising steps of:
   (a) under suitable conditions, culturing the host cell according to item 4, thereby obtaining a culture containing the bispecific antibody; and
   (b) purifying and/or separating the culture obtained in step (a) to obtain the bispecific antibody.
6. An immunoconjugate, characterized in that, the immunoconjugate comprises:
   (a) the bispecific antibody according to item 1; and
   (b) a coupling moiety selected from a group consisting of: detectable label, drug, toxin, cytokine, radionuclide, or enzyme, gold nanoparticle/nanorod, nanomagnetic particle, viral coat protein or VLP, or combination thereof.
7. Use of the bispecific antibody according to item 1 or the immunoconjugate according to item 6, in the manufacture of a medicament, a reagent, a detection plate or a kit; wherein, the reagent, detection plate or kit is used for: detecting PD-L1 and/or 4-1BB in a sample; wherein, the medicament is used for treating or preventing a tumor expressing PD-L1 (i.e., PD-L1 positive).
8. A pharmaceutical composition, characterized in that, the pharmaceutical composition comprises:
   (i) the bispecific antibody according to item 1, or the immunoconjugate according to item 6; and
   (ii) a pharmaceutically acceptable carrier.
9. One or more uses of the bispecific antibody according to item selected from the following group, comprising:
   (i) use for detecting human PD-L1 molecule and/or 4-1BB molecule; (ii) use for flow detection; (iii) use for cell immunofluorescence detection; (iv) use for treating a tumor; (v) use for diagnosis of a tumor; (vi) use for blocking the interaction between PD-1 and PD-L1; and (vii) use for binding to 4-1BB to activate an immune cell.
10. A recombinant protein, characterized in that, the recombinant protein comprises: (i) the bispecific antibody according to item 1; and (ii) an optional tag sequence that assists expression and/or purification.

The present invention further relates to any one of the following first to fourteenth aspects:
In the first aspect of the present invention, a bispecific antibody is provided, the bispecific antibody comprising:
(a) an anti-PD-L1 single-domain antibody; and
(b) an anti-4-1BB single-domain antibody.

In another preferred embodiment, the bispecific antibody comprises 1-3 anti-PD-L1 single domain antibodies, preferably, comprises 1 or 2 anti-PD-L1 single domain antibodies.

In another preferred embodiment, the PD-L1 single-domain antibody can block the interaction between PD-1 and PD-L1.

In another preferred embodiment, the bispecific antibody comprises 1-3 anti-4-1BB single domain antibodies, preferably, comprises 1 or 2 anti-4-1BB single domain antibodies.

In another preferred embodiment, the 4-1BB single-domain antibody can activate an immune cell.

In another preferred embodiment, the bispecific antibody further comprises an Fc region derived from a human immunoglobulin.

In another preferred embodiment, the human immunoglobulin is selected from a group consisting of IgG1, IgG2, IgG3, IgG4, or combination thereof; preferably IgG1.

In another preferred embodiment, the Fc region of the bispecific antibody is selected from a group consisting of: CH1+CL1 domain, human IgG domain or combination thereof.

In another preferred embodiment, the Fc region is an engineered mutant, preferably a LALA mutant and comprises a Knob-in-hole mutant.

In another preferred embodiment, the bispecific antibody is a dimer composed of peptide chain i and peptide chain ii, and the peptide chain i and peptide chain ii have structures shown in Formula I and Formula II, respectively,

A-L1-Fc1-L2-B (Formula I)

A-L3-Fc2-L4-B (Formula II)

wherein,
A and B are independently none, an anti-PD-L1 single-domain antibody or an anti-4-1BB single-domain antibody;
L1, L2 and L3 are each independently none or a linking element;
L4 is a linking element;
Fc1 and Fc2 are each independently an Fc region of a human immunoglobulin (preferably a LALA mutant); and
"-" represents a peptide bond;
and wherein, the bispecific antibody comprises at least one anti-PD-L1 single-domain antibody, and at least one anti-4-1BB single-domain antibody;
and wherein, the polypeptide represented by Formula I and the polypeptide represented by Formula II form a heterodimer through a disulfide bond interaction and a Knob-in-hole structure.

In another preferred embodiment, the bispecific antibody is a homodimer or a heterodimer.

In another preferred embodiment, the Fc region is a LALA mutant Fc, and has an amino acid sequence as set forth in SEQ ID NO: 3, or has ≥85% (preferably 90%, more preferably 95%) sequence identity to the SEQ ID NO: 3.

In another preferred embodiment, the Fc1 and Fc2 have knob mutation and hole mutation respectively.

In another preferred embodiment, in the amino acid sequence of Fc1, based on the amino acid sequence as set forth in SEQ ID NO: 3, the 132nd position has a S 132C mutation, and the 144^{th} position has a T144W mutation.

In another preferred embodiment, in the amino acid sequence of Fc2, based on the amino acid sequence as set forth in SEQ ID NO: 3, the 127^{th} position has a Y127C mutation, the 144^{th} position has a T144S mutation, and the 146^{th} position has a L146A mutation.

In another preferred embodiment, the Fc1 has an amino acid sequence as set forth in SEQ ID NO: 8, or has ≥85% (preferably 90%, more preferably 95%) sequence identity to the sequence set forth in SEQ ID NO: 8.

In another preferred embodiment, the Fc2 has an amino acid sequence as set forth in SEQ ID NO: 9, or has ≥85% (preferably 90%, more preferably 95%) sequence identity to the sequence set forth in SEQ ID NO: 9.

In another preferred embodiment, the anti-4-1BB single-domain antibody has an amino acid sequence as set forth in SEQ ID NO: 2, or has ≥85% (preferably 90%, more preferably 95%) sequence identity to the sequence as set forth in SEQ ID NO: 2.

In another preferred embodiment, the anti-PD-L1 single-domain antibody has an amino acid sequence as set forth in SEQ ID NO: 4, or has ≥85% (preferably 90%, more preferably 95%) sequence identity to the sequence as set forth in SEQ ID NO: 4.

In another preferred embodiment, the sequence of the linker is (G₄S)ₙ, wherein n is a positive integer (e.g., 1, 2, 3, 4, 5 or 6), preferably, n is 2 or 4.

In another preferred embodiment, the linker has an amino acid sequence as set forth in SEQ ID NO: 5, or has ≥85% (preferably 90%, more preferably 95%) sequence identity to the sequence set forth in SEQ ID NO: 5.

In another preferred embodiment, the bispecific antibody is a homodimer, which is formed by two identical peptide chains through a disulfide bond, and the peptide chains have an amino acid sequence as set forth in SEQ ID NO: 1 (i.e., Bi-400), or have ≥85% (preferably 90%, more preferably 95%) sequence identity to the sequence set forth in SEQ ID NO: 1.

In another preferred embodiment, the bispecific antibody is a homodimer, which is formed by two identical peptide chains through a disulfide bond, and the peptide chains have an amino acid sequence as set forth in SEQ ID NO: 6 (i.e., Bi-088), or have ≥85% (preferably 90%, more preferably 95%) sequence identity to the sequence set forth in SEQ ID NO: 6.

In another preferred embodiment, the bispecific antibody is a heterodimer, which is formed by peptide chain i and peptide chain ii through a Knob-in-hole interaction (Knob-in-hole); wherein, the peptide chain i has an amino acid sequence as set forth in SEQ ID NO: 7, or has ≥ 85% (preferably 90%, more preferably 95%) sequence identity to the sequence set forth in SEQ ID NO: 7; and the peptide chain ii has an amino acid sequence as set forth in SEQ ID NO: 9, or has ≥85% (preferably 90%, more preferably 95%) sequence identity to the sequence set forth in SEQ ID NO: 9; (i.e., Bi-401-091).

In another preferred embodiment, the bispecific antibody is a heterodimer, which is formed by peptide chain i and peptide chain ii through a Knob-in-hole interaction (Knob-in-hole); wherein, the peptide chain i has an amino acid sequence as set forth in SEQ ID NO: 7, or has ≥ 85% (preferably 90%, more preferably 95%) sequence identity to the sequence set forth in SEQ ID NO: 7; and the peptide chain ii has an amino acid sequence as set forth in SEQ ID NO: 10, or has ≥85% (preferably 90%, more preferably 95%) sequence identity to the sequence set forth in SEQ ID NO: 10; (i.e., Bi-061-091).

In the second aspect of the present invention, an isolated polynucleotide is provided, and the isolated polynucleotide encodes the bispecific antibody according to the first aspect of the present invention.

In another preferred embodiment, when the bispecific antibody is a heterodimer, in the polynucleotide, the polynucleotide sequence encoding the peptide chain i and the polynucleotide sequence encoding the peptide chain ii are in a ratio of 1:1.

In the third aspect of the present invention, a vector is provided, and the vector comprises the polynucleotide according to the second aspect of the present invention.

In another preferred embodiment, the vector is selected from a group consisting of: DNA, RNA, viral vector, plasmid, transposon, other gene transfer system, or combination thereof; preferably, the expression vector includes viral vector, such as Lentivirus, adenovirus, AAV virus, retrovirus, or combination thereof.

In the fourth aspect of the present invention, a host cell is provided, the host cell comprises the vector according to the third aspect of the present invention, or the polynucleotide according to the second aspect of the present invention integrated into its genome;
or, the host cell expresses the bispecific antibody according to the first aspect of the present invention.

In another preferred embodiment, the host cell comprises prokaryotic cell or eukaryotic cell.

In another preferred embodiment, the host cell is selected from a group consisting of: *Escherichia coli,* yeast cell, and mammalian cell.

In the fifth aspect of the present invention, there is provided a method for producing the bispecific antibody according to the first aspect of the present invention, comprising steps of:
(a) cultivating the host cell according to the fourth aspect of the present invention under suitable conditions, so as to obtain a culture containing the bispecific antibody; and
(b) purifying and/or separating the culture obtained in step (a) to obtain the bispecific antibody.

In another preferred embodiment, the purification can be performed to the target antibody through protein A affinity column purification and separation.

In another preferred embodiment, the purified target antibody has a purity of greater than 95%, greater than 96%, greater than 97%, greater than 98%, greater than 99%, preferably 100%.

In the sixth aspect of the present invention, an immunoconjugate is provided, which comprises:
(a) the bispecific antibody according to the first aspect of the present invention; and
(b) a coupling moiety selected from a group consisting of: detectable label, drug, toxin, cytokine, radionuclide, or enzyme, gold nanoparticle/nanorod, nanomagnetic particle, viral coat protein or VLP, or combination thereof.

In another preferred embodiment, the radionuclide comprises:
(i) an isotope for diagnosis, in which the isotope for diagnosis is selected from a group consisting of: Tc-99m, Ga-68, F-18, I-123, I-125, I-131, In-111, Ga-67, Cu-64, Zr-89, C-11, Lu-177, Re-188, or combination thereof; and/or
(ii) a therapeutic isotope, in which the therapeutic isotope is selected from a group consisting of: Lu-177, Y-90, Ac-225, As-211, Bi-212, Bi-213, Cs-137, Cr-51, Co-60, Dy-165, Er-169, Fm-255, Au-198, Ho-166, I-125, 1-131, Ir-192, Fe-59, Pb-212, Mo-99, Pd- 103, P-32, K-42, Re-186, Re-188, Sm-153. Ra223, Ru-106, Na24, Sr89, Tb-149, Th-227, Xe-133 Yb-169, Yb-177, or combination thereof.

In another preferred embodiment, the coupling moiety is a drug or a toxin.

In another preferred embodiment, the drug is a cytotoxic drug.

In another preferred embodiment, the cytotoxic drug is selected from a group consisting of: anti-tubulin drug, DNA minor groove binding agent, DNA replication inhibitor, alkylating agent, antibiotic, folic acid antagonist, antimetabolite, chemotherapy A sensitizer, topoisomerase inhibitor, vinca alkaloid, or combination thereof.

Examples of particularly useful classes of cytotoxic drugs include, for example, DNA minor groove binding agent, DNA alkylating agent, and tubulin inhibitor. Typical cytotoxic drugs include, for example, auristatins, camptothecins, duocarmycins, etoposides, maytansines and maytansinoids (e.g., DM1 and DM4), taxanes, benzodiazepines, or benzodiazepine-containing drugs (e.g., pyrrolo[1,4]benzodiazepines (PBDs), indolinobenzodiazepines and oxazolidinobenzodiazepines, vinca alkaloids, or combinations thereof.

In another preferred embodiment, the toxin is selected from a group consisting of:
auristatins (e.g., auristatin E, auristatin F, MMAE, and MMAF), aureomycin, maytansinoids, ricin, ricin A-chain, combretastatin, duocarmycin, dolastatin, doxorubicin, daunorubicin, paclitaxel, cisplatin, cc1065, ethidium bromide, mitomycin, etoposide, tenoposide, vincristine, vinblastine, colchicine, dihydroxyanthracindione, actinomycin, diphtheria toxin, Pseudomonas exotoxin (PE) A, PE40, abrin, abrin A chain, modeccin A chain, α -Sarcina, gelonin, mitogellin, retstrictocin, phenomycin, enomycin, curicin, crotin, calicheamicin, Sapaonaria officinalis inhibitor, glucocorticoid, or combination thereof.

In another preferred embodiment, the coupling moiety is a detectable label.

In another preferred embodiment, the conjugate is selected from a group consisting of: fluorescent or luminescent label, radioactive label, MRI (magnetic resonance imaging) or CT (computer X-ray tomography) contrast agent, or enzyme capable of producing detectable product, radionuclide, biotoxin, cytokine (e.g., IL-2), antibody, antibody Fc fragment, antibody scFv fragment, gold nanoparticle/nanorod, virus particle, liposome, nanomagnetic particle, prodrug-activating enzyme (e.g., DT-diaphorase (DTD) or biphenylhydrolase-like protein (BPHL)), chemotherapeutic agent (e.g., cisplatin).

In another preferred embodiment, the immunoconjugate comprises: a multivalent (e.g., bivalent) bispecific antibody as described in the first aspect of the present invention.

In another preferred embodiment, the multivalent refers to that the immunoconjugate has an amino acid sequence comprising multiple repeats of the bispecific antibody according to the first aspect of the present invention.

In the seventh aspect of the present invention, there is provided a use of the bispecific antibody as described in the first aspect of the present invention, or the immunoconjugate as described in the sixth aspect of the present invention in the manufacture of a medicament, a reagent, a detection plate or kit; wherein, the reagent, detection plate or kit is used for: detecting PD-L1 and/or 4-1BB in a sample; wherein, the medicament is used for treating or preventing a tumor expressing PD-L1 (i.e., PD-L1 positive).

In another preferred embodiment, the coupling moiety of the immunoconjugate is an isotope for diagnosis.

In another preferred embodiment, the reagent is one or more reagents selected from a group consisting of: isotopic tracer, contrast agent, flow detection reagent, cellular immunofluorescence detection reagent, magnetic nanoparticle and imaging agent.

In another preferred embodiment, the reagent for detecting PD-L1 and/or 4-1BB in the sample is a contrast agent for detecting PD-L1 and/or 4-1BB molecules (in vivo).

In another preferred embodiment, the detection is an in vivo detection or an in vitro detection.

In another preferred embodiment, the detection comprises flow detection and cell immunofluorescence detection.

In another preferred embodiment, the agent is used for blocking an interaction between PD-1 and PD-L1, and at the same time activating an immune cell by binding to 4-1BB.

In another preferred embodiment, the tumor comprises but is not limited to: acute myeloid leukemia, chronic myelocytic leukemia, multiple myelopathy, non-Hodgkin's lymphoma, colorectal cancer, breast cancer, colorectal cancer, gastric cancer, liver cancer, leukemia, kidney tumor, lung cancer, small bowel cancer, bone cancer, prostate cancer, prostate cancer, cervical cancer, lymphoma, adrenal tumor, bladder tumor, or combination thereof.

In the eighth aspect of the present invention, a pharmaceutical composition is provided, which comprises:
(i) the bispecific antibody as described in the first aspect of the present invention, or the immunoconjugate as described in the sixth aspect of the present invention; and
(ii) a pharmaceutically acceptable carrier.

In another preferred embodiment, the coupling moiety of the immunoconjugate is a drug, a toxin, and/or a therapeutic isotope.

In another preferred embodiment, the pharmaceutical composition further comprises an additional drug such as cytotoxic drug for treating a tumor.

In another preferred embodiment, the additional drug for treating tumor comprises paclitaxel, doxorubicin, cyclophosphamide, axitinib, lenvatinib, and pembrolizumab.

In another preferred embodiment, the pharmaceutical composition is used for treating a tumor expressing PD-L1 protein (i.e., PD-L1 positive).

In another preferred embodiment, the pharmaceutical composition is in the form of injection.

In another preferred embodiment, the pharmaceutical composition is used in the manufacture of a medicament for preventing and treating a tumor.

In the ninth aspect of the present invention, there is provided one or more uses of the bispecific antibody according to the first aspect of the present invention selected from the following group, which comprises:
(i) use for detecting human PD-L1 molecule and/or 4-1BB molecule; (ii) use for flow detection; (iii) use for cell immunofluorescence detection; (iv) use for treating a tumor; (v) use for diagnosis of a tumor; (vi) use for blocking an interaction between PD-1 and PD-L1; and (vii) use for binding to 4-1BB to activate an immune cell.

In another preferred embodiment, the tumor is a tumor expressing PD-L1 protein (i.e., PD-L1 positive).

In another preferred embodiment, the use is non-diagnostic and non-therapeutic.

In the tenth aspect of the present invention, a recombinant protein is provided, which has: (i) the bispecific antibody according to the first aspect of the present invention; and (ii) optionally a tag sequence for assisting expression and/or or purification.

In another preferred embodiment, the tag sequence comprises 6His tag, HA tag and Fc tag.

In another preferred embodiment, the recombinant protein specifically binds to PD-L1 and/or 4-1BB.

In the eleventh aspect of the present invention, there is provided a method for detecting PD-L1 and/or 4-1BB in a sample, and the method comprises the steps of: (1) contacting the sample with the bispecific antibody as described in the first aspect of the present invention; (2) detecting whether an antigen-antibody complex is formed, wherein the formation of the complex indicates the presence of PD-L1 and/or 4-1BB in the sample.

In the twelfth aspect of the present invention, there is provided a method for treating a disease, and the method comprises: administering the bispecific antibody as described in the first aspect of the present invention, or the immunoconjugate described in the sixth aspect of the present invention, or the pharmaceutical composition as described in the eighth aspect of the present invention to a subject in need thereof.

In another preferred embodiment, the subject comprises mammal, preferably human.

In the thirteenth aspect of the present invention, there is provided a detection reagent for PD-L1 and/or 4-1BB, characterized in that, the detection reagent comprises the immunoconjugate as described in the sixth aspect of the present invention and a detection-acceptable carrier.

In another preferred embodiment, the coupling moiety of the immunoconjugate is an isotope for diagnosis.

In another preferred embodiment, the detection-acceptable carrier is a non-toxic, inert aqueous carrier medium.

In another preferred embodiment, the detection reagent is one or more reagents selected from a group consisting of: isotopic tracer, contrast agent, flow detection reagent, cellular immunofluorescence detection reagent, magnetic nanoparticle and imaging agent.

In another preferred embodiment, the detection reagent is used for in vivo detection.

In another preferred embodiment, the dosage form of the detection reagent is liquid or powder (e.g., aqueous solution, injection, freeze-dried powder, tablet, buccal preparation, aerosol).

In the fourteenth aspect of the present invention, a kit for detecting PD-L1 and/or 4-1BB is provided, characterized in that the kit comprises the immunoconjugate as described in the sixth aspect of the present invention or the detection reagent as described in the thirteenth aspect of the present invention, and an instruction.

In another preferred embodiment, the instruction describes that the kit is used for a non-invasive detection of the expression of PD-L1 and/or 4-1BB in a subject.

In another preferred embodiment, the kit is used for a detection of a tumor expressing PD-L1 protein (i.e., PD-L1 positive).

### Terminology

In order that the present disclosure may be more readily understood, certain terms are first defined. As used in this application, unless expressly stated otherwise herein, each of the following terms shall have the meaning given below. Other definitions are set forth throughout the present application.

As used herein, the term "about" can refer to a value or composition within an acceptable error range for a particular value or composition as determined by one of ordinary skill in the art, which will depend in part on how the value or composition is measured or determined.

As used herein, the terms "give" and "administrate" are used interchangeably and refer to that the product of the present invention is physically introduced into a subject using any of a variety of methods and delivery systems known to those skilled in the art, including intravenous, intramuscular, subcutaneous, intraperitoneal, spinal or other parenteral routes of administration, for example by injection or infusion.

### Bispecific antibody

As used herein, the terms "bispecific antibody of the present invention", "double antibody of the present invention", and "anti-PD-L1/4-1BB bispecific antibody" have the same meaning, and they all refer to a bispecific antibody capable of specifically recognizing and binding to PD-L1 and 4-1BB.

The present invention provides an anti-PD-L1/4-1BB bispecific antibody, which comprises: an anti-PD-L1 single-domain antibody and an anti-4-1BB single-domain antibody.

Preferably, the bispecific antibody is a dimer composed of peptide chain i and peptide chain ii, and the structures of peptide chain i and peptide chain ii are shown in Formula I and Formula II respectively,

A-L1-Fc1-L2-B (Formula I)

A-L3-Fc2-L4-B (Formula II)

wherein,
A and B are independently none, anti-PD-L1 single-domain antibody or anti-4-1BB single-domain antibody;
L1, L2 and L3 are each independently none or a linker;
L4 is a linker;
Fc1 and Fc2 are each independently an Fc region of a human immunoglobulin (preferably a LALA mutant); and
"-" represents a peptide bond;
and wherein, the bispecific antibody comprises at least one anti-PD-L1 single-domain antibody, and at least one anti-4-1BB single-domain antibody;
and wherein, the polypeptide represented by Formula I and the polypeptide represented by Formula II form a heterodimer through a disulfide bond interaction and a knob-in-hole structure (Knob-in-hole).

In an embodiment of the present invention, the bispecific antibody is a homodimer or a heterodimer.

In one embodiment, the Fc region is a LALA mutant Fc, and has an amino acid sequence as set forth in SEQ ID NO: 3, or has ≥85% (preferably 90%, more preferably 95%) sequence identity to the sequence set forth in SEQ ID NO: 3.

In another embodiment, the Fc1 and Fc2 have a knob mutation and a hole mutation, respectively. Wherein, the knob mutation refers to that, based on the amino acid sequence set forth in SEQ ID NO: 3, there are an S132C mutation at position 132 and a T144W mutation at position 144. The hole mutation refers to that based on the amino acid sequence set forth in SEQ ID NO: 3, there are a Y127C mutation at position 127, a T144S mutation at position 144, and a L146A mutation at position 146.

Preferably, the Fc1 has an amino acid sequence as set forth in SEQ ID NO: 8, or has ≥ 85% (preferably 90%, more preferably 95%) sequence identity to the sequence set forth in SEQ ID NO: 8; the Fc2 has an amino acid sequence as set forth in SEQ ID NO: 9, or has ≥85% (preferably 90%, more preferably 95%) sequence identity to the sequence set forth in SEQ ID NO: 9.

As used herein, the terms "single-domain antibody", "nanobody VHH", and "nanobody" have the same meaning and refer to a nanobody (VHH) consisting of only one heavy chain variable region constructed by cloning an antibody heavy chain variable region, which is the smallest antigen-binding fragment with complete function. Usually, after obtaining the antibody that naturally lacks the light chain and heavy chain constant region 1 (CH1), the antibody heavy chain variable region is cloned to construct a nanobody (VHH) consisting of only one heavy chain variable region.

As used herein, the term "variable" means that certain portions of antibody variable regions differ in sequence, which contribute to the binding and specificity of each particular antibody for its particular antigen. However, the variability is not evenly distributed throughout antibody variable domains. It is concentrated in three segments called complementarity determining regions (CDRs) or hypervariable regions in the light chain and heavy chain variable regions. The more conserved portions of the variable domains are called framework regions (FRs). The variable regions of native heavy and light chains each contain four FR regions that are in a roughly-folded configuration and connected by three CDRs that form joining loops, which in some cases may form a partially folded structure. The CDRs in each chain are brought into close proximity by the FR regions and together with the CDRs of the other chain form the antigen-binding site of the antibody (see Kabat et al., NIH Publ. No. 91-3242, Vol. I, pp. 647-669 (1991)). The constant regions are not directly involved in the binding of antibody to antigen, but they exhibit different effector functions, for example being involved in the antibody-dependent cytotoxicity of antibody.

As used herein, the term "framework region" (FR) refers to an amino acid sequence inserted between CDRs, i.e. a portion of immunoglobulin light and heavy chain variable regions that is relatively conservative among different immunoglobulins in a single species. Each of immunoglobulin light and heavy chains has four FRs, referred to as FR1-L, FR2-L, FR3-L, FR4-L and FR1-H, FR2-H, FR3-H, FR4-H, respectively. Accordingly, the light chain variable domain may thus be referred to as (FR1-L)-(CDR1-L)-(FR2-L)-(CDR2-L)-(FR3-L)-(CDR3-L)-(FR4-L) and the heavy chain variable domain can thus be expressed as (FR1-H)-(CDR1-H)-(FR2-H)-(CDR2-H)-(FR3-H)-(CDR3-H)-(FR4-H). Preferably, the FR of the present invention is a human antibody FR or a derivative thereof, and the human antibody FR derivative is substantially identical to the naturally occurring human antibody FR, that is, the sequence identity reaches 85%, 90%, 95%, or 96%., 97%, 98%, or 99%.

Knowing the amino acid sequences of CDRs, those skilled in the art can easily determine the framework regions FR1-L, FR2-L, FR3-L, FR4-L and/or FR1-H, FR2-H, FR3-H, FR4-H.

As used herein, the term "human framework region" is a framework region that is substantially (about 85% or more, specifically 90%, 95%, 97%, 99% or 100%) identical to a naturally occurring human antibody framework region.

As used herein, the term "affinity" is theoretically defined by the equilibrium association between intact antibody and antigen. The affinity of the bispecific antibody of the present invention can be evaluated or determined by K_{D} value (dissociation constant) or other measurement methods, for example, determined by bio-layer interferometry (BLI) using FortebioRed96 instrument.

As used herein, the term "linker" refers to one or more amino acid residues inserted into immunoglobulin domain to provide sufficient mobility for the domains of light and heavy chains to fold into an immunoglobulin with exchanged dual variable regions.

As known to those skilled in the art, immunoconjugates and fusion expression products include conjugates formed by binding a drug, toxin, cytokine, radionuclide, enzyme and other diagnostic or therapeutic molecules to the antibody or fragment thereof of the present invention. The present invention also comprises a cell surface marker or antigen that binds to the PD-L1/4-1BB bispecific antibody or fragment thereof.

As used herein, the terms "variable region" and "complementarity determining region (CDR)" are used interchangeably.

In a preferred embodiment of the present invention, the heavy chain variable region of the antibody comprises three complementarity determining regions, CDR1, CDR2 and CDR3.

In a preferred embodiment of the present invention, the heavy chain of the antibody comprises the above-mentioned heavy chain variable region and heavy chain constant region.

In the present invention, the terms "antibody of the present invention", "protein of the present invention", or "polypeptide of the present invention" are used interchangeably, and all refer to a polypeptide that specifically binds to PD-L1 and/or 4-1BB protein, for example, a protein or polypeptide having the heavy chain variable regions. They may or may not contain starting methionine.

The present invention further provides another protein or fusion expression product having the antibody of the present invention. Specifically, the present invention comprises any protein or protein conjugate and fusion expression product (i.e., immunoconjugate and fusion expression product) having a heavy chain containing a variable region, as long as the variable region is identical or at least 90% homologous, preferably at least 95% homologous to the heavy chain variable region of the antibody of the present invention.

In general, the antigen-binding properties of an antibody can be described by 3 specific regions located in a heavy chain variable region, which are called CDRs and separated the segment into 4 framework regions (FRs), and the amino acid sequences of the 4 FRs are relatively conservative and do not directly participate in the binding reaction. These CDRs form a ring structure, and the β folds formed by the FRs therebetween are close to each other in the spatial structure, and the CDRs on heavy chain and the corresponding CDRs on light chain constitute the antigen-binding site of the antibody. By comparing the amino acid sequences of antibodies of the same type, it is possible to determine which amino acids constitute FR or CDR regions.

The variable regions of the heavy chains of the antibody of the present invention are of particular interest, because at least some of them are involved in the binding to antigen. Therefore, the present invention comprises those molecules having an antibody heavy chain variable region with a CDR, as long as the CDR is more than 90% (preferably more than 95%, and most preferably more than 98%) homologous to the CDR identified herein.

The present invention comprises not only an intact antibody, but also an antibody fragment with immunological activity or a fusion protein formed with the antibody and other sequences. Accordingly, the present invention further comprises fragments, derivatives and analogs of the antibody.

As used herein, the terms "fragment", "derivative" and "analogue" refer to a polypeptide that substantially retains the same biological function or activity of the antibody of the present invention. The polypeptide fragment, derivative or analog of the present invention may be (i) a polypeptide having one or more substituted conservative or non-conservative amino acid residues (preferably conservative amino acid residues), and such substituted amino acid residues may or may not be encoded by genetic code, or (ii) a polypeptide having a substituent group in one or more amino acid residues, or (iii) a polypeptide formed by fusing a mature polypeptide to another compound (e.g., a compound that extends the half-life of the polypeptide, for example, polyethylene glycol), or (iv) a polypeptide formed by fusing an additional amino acid sequence to a polypeptide sequence (e.g., a fusion protein formed by a leader sequence or a secretory sequence or a sequence or proprotein sequence for purifying the polypeptide, or by a 6His tag). In light of the teachings herein, such fragment, derivative and analog are within the purview of those skilled in the art.

The antibody of the present invention refers to a double antibody with activity of binding to PD-L1 and/or 4-1BB protein. The term also refers to a polypeptide variant comprising the same CDR regions and having the same function as the antibody of the present invention. Such variant form includes (but is not limited to): deletion, insertion and/or substitution of one or more (usually 1-50, preferably 1-30, more preferably 1-20, and most preferably 1-10) amino acids, and addition of one or several (usually within 20, preferably within 10, more preferably within 5) amino acids at C-terminal and/or N-terminal. For example, in the art, substitution of amino acid with closer or similar properties generally does not change the function of protein. As another example, addition of one or several amino acids at C-terminal and/or N-terminal usually does not change the function of protein. The term further refers to an active fragment and active derivative of the antibody of the present invention.

Variants of the polypeptide include: homologous sequence, conservative variant, allelic variant, natural mutant, induced mutant, protein encoded by a DNA capable of hybridizing to the DNA of the antibody of the present invention under highly or lowly stringent conditions, and polypeptide or protein obtained by using the antiserum against the antibody of the present invention.

The present invention further provides other polypeptides, such as fusion proteins comprising single domain antibodies or fragments thereof. In addition to substantially full-length polypeptides, the present invention further comprises fragments of the single domain antibodies of the present invention. Typically, the fragment has at least about 50 contiguous amino acids, preferably at least about 50 contiguous amino acids, more preferably at least about 80 contiguous amino acids, and most preferably at least about 100 contiguous amino acids of the antibody of the present invention.

In the present invention, "conservative variant of the antibody of the present invention" refers to a polypeptide formed by substitution of at most 10, preferably at most 8, more preferably at most 5, and most preferably at most 3 amino acid sequences with amino acids with similar or closer properties as compared with the amino acid sequence of the antibody of the present invention. These conservative variant polypeptides are preferably produced by amino acid substitutions according to Table A.

**Table A**

| Original residue | Representative substitution | Preferred substitution |
|---|---|---|
| Ala (A) | Val; Leu; Ile | Val |
| Arg (R) | Lys; Gln; Asn | Lys |
| Asn (N) | Gln; His; Lys; Arg | Gln |
| Asp (D) | Glu | Glu |
| Cys (C) | Ser | Ser |
| Gln (Q) | Asn | Asn |
| Glu (E) | Asp | Asp |
| Gly (G) | Pro; Ala | Ala |
| His (H) | Asn; Gin; Lys; Arg | Arg |
| Ile (I) | Leu; Val; Met; Ala; Phe | Leu |
| Leu (L) | Ile; Val; Met; Ala; Phe | Ile |
| Lys (K) | Arg; Gln; Asn | Arg |
| Met (M) | Leu; Phe; Ile | Leu |
| Phe (F) | Leu; Val; Ile; Ala; Tyr | Leu |
| Pro (P) | Ala | Ala |
| Ser (S) | Thr | Thr |
| Thr (T) | Ser | Ser |
| Trp (W) | Tyr; Phe | Tyr |
| Tyr (Y) | Trp; Phe; Thr; Ser | Phe |
| Val (V) | Ile; Leu; Met; Phe; Ala | Leu |

The present invention further provides a polynucleotide molecule encoding the above-mentioned antibody or fragment thereof or fusion protein thereof. The polynucleotide of the present invention may be in the form of DNA or RNA. The form of DNA comprises cDNA, genomic DNA or synthetic DNA. The DNA can be single-stranded or double-stranded. The DNA can be either a coding strand or a non-coding strand.

The polynucleotide encoding the mature polypeptide of the present invention comprises: a coding sequence that encodes only the mature polypeptide; a coding sequence for the mature polypeptide and various additional coding sequences; a coding sequence for the mature polypeptide (and optional additional coding sequences) and non-coding sequences.

The term "polynucleotide encoding polypeptide" may include a polynucleotide encoding the polypeptide, or may also include an additional coding and/or non-coding sequence.

The present invention further relates to a polynucleotide that hybridizes to the above-mentioned sequence and has at least 50%, preferably at least 70%, more preferably at least 80% identity between the two sequences. The present invention particularly relates to a polynucleotide which is hybridizable under stringent conditions to the polynucleotide of the present invention. In the present invention, "stringent conditions" refer to: (1) hybridization and elution at lower ionic strength and higher temperature, for example, 0.2×SSC, 0.1% SDS, 60°C; or (2) hybridization in the presence of a denaturing agent, for example, 50% (v/v) formamide, 0.1% calf serum/0.1% Ficoll, 42°C, etc.; or (3) hybridization that occurs only when the identity between two sequences is at least 90%, preferably more than 95%. Moreover, the polypeptide encoded by the hybridizable polynucleotide has the same biological function and activity as the mature polypeptide.

The full-length nucleotide sequence of the antibody of the present invention or its fragment can usually be obtained by PCR amplification, recombination or artificial synthesis. A feasible method is to use artificial synthesis to synthesize related sequences, especially when the fragment length is short. Usually, a fragment with very long sequence is obtained by synthesizing multiple small fragments and then ligating them. In addition, a fusion protein may also be formed by fusing the coding sequence of heavy chain with an expression tag (e.g., 6His).

Once a relevant sequence is obtained, a recombinant method can be used to obtain the relevant sequences in large quantity. Usually, it is cloned into a vector, then transformed into a cell, and then the relevant sequence is isolated from the proliferated host cell by conventional methods. The biomolecules (nucleic acid, protein, etc.) involved in the present invention include biomolecules in isolated form.

At present, the DNA sequence encoding the protein of the present invention (or its fragment, or its derivative) can be obtained completely through chemical synthesis. The DNA sequence can then be introduced into various existing DNA molecules (e.g., vectors) and cells known in the art. In addition, a mutation can also be introduced into the protein sequence of the present invention by chemical synthesis.

The present invention also relates to a vector comprising the above-mentioned appropriate DNA sequence and appropriate promoter or control sequence. These vectors can be used to transform appropriate host cells so that they express the protein.

The host cell may be a prokaryotic cell, such as bacterial cell; or lower eukaryotic cell, such as yeast cell; or a higher eukaryotic cell, such as a mammalian cell. Representative examples are: *Escherichia coli, Streptomyces;* bacterial cell such as *Salmonella typhimurium;* fungal cell such as yeast; insect cell such as *Drosophila* S2 or Sf9; animal cell such as CHO, COS7, 293 cell, etc.

Transformation of host cells with recombinant DNA can be performed using conventional techniques well known to those skilled in the art. When the host is a prokaryotic organism such as *Escherichia coli,* competent cells capable of taking up DNA can be harvested after the exponential growth phase and treated with CaCl₂ using procedures well known in the art. Another way is to use MgCl₂. The transformation can also be performed by electroporation, if desired. When the host is a eukaryotic organism, the following DNA transfection methods can be used: calcium phosphate co-precipitation method, conventional mechanical methods such as microinjection, electroporation, liposome packaging, etc.

The obtained transformant can be cultured by conventional methods to express the polypeptide encoded by the gene of the present invention. The medium used in the culture can be selected from various conventional media according to the host cells as used. The culture is carried out under conditions suitable for the growth of the host cell. After the host cell has grown to an appropriate cell density, the selected promoter is induced by an appropriate method (e.g., temperature shift or chemical induction), and the cells are cultured for an additional period of time.

The recombinant polypeptide in the above method can be expressed inside the cell, or on the cell membrane, or secreted outside the cell. The recombinant protein can be isolated and purified by various separation methods by taking advantage of its physical, chemical and other properties, if desired. These methods are well known to those skilled in the art. Examples of these methods include, but are not limited to: conventional renaturation treatment, treatment with protein precipitating agent (salting out method), centrifugation, osmotic disruption, supertreatment, ultracentrifugation, molecular sieve chromatography (gel filtration), adsorption chromatography, ion exchange chromatography, high performance liquid chromatography (HPLC) and various other liquid chromatography techniques and combinations of these methods.

The antibody of the present invention can be used alone, or combined or conjugated with a detectable label (for diagnostic purposes), a therapeutic agent, a PK (protein kinase) modifying moiety, or a combination of any of these substances.

The detectable label for diagnostic purposes includes, but is not limited to, fluorescent or luminescent label, radioactive label, MRI (magnetic resonance imaging) or CT (computed tomography) contrast agent, or enzyme capable of producing a detectable product.

The therapeutic agent that can be combined or conjugated with the antibody of the present invention includes, but is not limited to: 1. radionuclide; 2. biological toxicity; 3. cytokine such as IL-2, etc.; 4. gold nanoparticle/nanorod; 5. virus; 6. liposome; 7. nanomagnetic particle; 8. prodrug-activating enzyme (e.g., DT-diaphorase (DTD) or biphenylhydrolase-like protein (BPHL)); 10. chemotherapeutic agent (for example, cisplatin) or any form of nanoparticle, etc.

### Pharmaceutical composition

The present invention also provides a composition. Preferably, the composition is a pharmaceutical composition, which comprises the above-mentioned antibody or its active fragment or its fusion protein, and a pharmaceutically acceptable carrier. Generally, these materials can be formulated in a non-toxic, inert and pharmaceutically acceptable aqueous carrier medium, wherein the pH is usually about 5-8, preferably about 6-8, although the pH value can be changed according to the nature of the substances formulated and the conditions to be treated. The prepared pharmaceutical composition can be administered by conventional routes, including (but not limited to): intratumoral, intraperitoneal, intravenous, or topical administration.

The pharmaceutical composition of the present invention can be directly used for binding PD-L1 and/or 4-1BB protein molecules, and thus can be used for treating a tumor. In addition, an additional therapeutic agent may also be used concomitantly.

The pharmaceutical composition of the present invention comprises a safe and effective amount (e.g., 0.001-99wt%, preferably 0.01-90wt%, more preferably 0.1-80wt%) of the above-mentioned single-domain antibody (or its conjugate) of the present invention and a pharmaceutical acceptable carrier or excipient. Such carrier includes, but is not limited to: saline, buffer, dextrose, water, glycerol, ethanol, and combination thereof. The pharmaceutical formulation should match the mode of administration. The pharmaceutical composition of the present invention can be prepared in the form of injection, for example, by conventional methods using physiological saline or aqueous solution containing glucose and other adjuvants. The pharmaceutical composition such as injection or solution is preferably produced under sterile conditions. The active ingredient is administered in a therapeutically effective amount, for example about 10 µg/kg body weight to about 50 mg/kg body weight per day. In addition, the polypeptide of the present invention can also be used with an additional therapeutic agent.

When using the pharmaceutical composition, a safe and effective amount of the immunoconjugate is administered to a mammal, wherein the safe and effective amount is usually at least about 10 µg/kg body weight, and in most cases no more than about 50 mg/kg body weight; preferably, the dosage is about 10 µg/kg body weight to about 10 mg/kg body weight. Nevertheless, factors such as the route of administration and the health status of the patient should also be considered for the specific dosage, which are within the skill of skilled physicians.

### Labeled antibody

In a preferred embodiment of the present invention, the antibody bears a detectable label. More preferably, the label is selected from a group consisting of isotope, colloidal gold label, colored label or fluorescent label.

Colloidal gold labeling can be performed using methods known to those skilled in the art. In a preferred embodiment of the present invention, the PD-L1/4-1BB bispecific antibody can be labeled with a colloidal gold to obtain a colloidal gold-labeled antibody.

### Detection method

The present invention also relates to a method for detecting PD-L1 and/or 4-1BB proteins. The method roughly comprises the following steps: obtaining a sample of cell and/or tissue; dissolving the sample in a medium; detecting the level of PD-L1 and/or 4-1BB proteins in the dissolved sample.

In the detection method of the present invention, the sample used is not particularly limited, and a representative example is a cell-containing sample present in a cell preservation solution.

### Kit

The present invention also provides a kit comprising the antibody (or its fragment) or detection plate of the present invention. In a preferred embodiment of the present invention, the kit further comprises a container, an instruction for use, a buffer, etc.

The present invention also provides a detection kit for detecting the level of PD-L1 and/or 4-1BB, and the kit comprises an antibody for recognizing PD-L1 and/or 4-1BB protein, a lysis medium for dissolving sample, and common detection reagents and buffers such as various buffers, detection labels, detection substrates, etc. The detection kit may be an in vitro diagnostic device.

### Use

As mentioned above, the single-domain antibody of the present invention has a wide range of biological and clinical application values, and its use involves many fields such as the diagnosis and treatment of a disease related to PD-L1 and/or 4-1BB, basic medical research, biological research, etc. A preferred use thereof is for clinical diagnosis and targeted therapy against PD-L1 and/or 4-1BB, such as tumor therapy.

### Beneficial effects of the present invention

The present invention achieves any one or more of the following technical effects:
1) The nanobody of the present invention is highly specific for human PD-L1 protein and 4-1BB protein with correct spatial structure.
2) The bispecific antibody of the present invention has strong affinity.
3) The production of the bispecific antibody of the present invention is simple and convenient.
4) The bispecific antibody of the present invention has the function of blocking the binding of PD-L1 to the receptor PD-1. At the same time, the bispecific antibody can also bind to 4-1BB on immune cells, activate the activity of immune cells in the tumor microenvironment, and more effectively improve the effect of inhibiting tumor occurrence and development.
5) The bispecific antibody has good stability and long half-life.
6) It has little hepatotoxicity and good safety.
7) There is very likely to be a synergistic effect between the first protein functional region and the second protein functional region of the bispecific antibody of the present invention, for example: its ability to block the binding of PD-L1 to PD-1 is even better than that of the control antibody; the level of binding to human 4-1BB and human PD-L1 protein is even better than that of the control antibody; it is also better than the control antibody in terms of inducing mixed lymphocytes to secrete IL-2, etc., indicating that the bispecific antibody of the present invention can better activate T cells, and so on.

### Brief Description of the Drawings

Figure 1A to Figure 1D show schematic diagrams of the structures of bispecific antibodies Bi-400, Bi-088, Bi-401-091 and Bi-061-091, respectively. The meanings of each module are as follows: 
4-1BB sdAb
PD-L1 sdAb
Fc
Fc-knob
Fc-hole
(G4S)4 linker
.
Figure 2A shows the detection results of the binding of the bispecific antibody of the present invention to the overexpressed human PD-L1 protein on the surface of CHO-S cells.
Figure 2B shows the detection results of the binding of the bispecific antibody of the present invention to the overexpressed cynomolgus monkey PD-L1 protein on the surface of CHO-S cells.
Figure 2C shows the detection results of the binding of the bispecific antibody of the present invention to the human 4-1BB protein overexpressed on the surface of CHO-S cells.
Figure 2D shows the detection results of the binding of the bispecific antibody of the present invention to the overexpressed cynomolgus monkey 4-1BB protein on the surface of CHO-S cells.
Figure 2E shows the detection results of the binding of the bispecific antibody of the present invention to CHO-S cells.
Figure 3 shows the detection results of the bispecific antibody of the present invention blocking the binding of PD-L1 protein to human PD-1 protein overexpressed on the surface of CHO-S cells.
Figure 4A shows the detection results of the bispecific antibody of the present invention simultaneously binding to the CHO-S cells overexpressing human 4-1BB and the CHO-S cells overexpressing human PD-L1.
Figure 4B shows the detection results of the bispecific antibody of the present invention simultaneously binding to human 4-1BB and human PD-L1 proteins.
Figure 5A shows the detection results of the fluorescent signal activation activity of the bispecific antibody of the present invention in the co-incubation system of CHO-S cells and cells overexpressing human 4-1BB Jurkat NF-AT luciferase reporter gene.
Figure 5B shows the detection results of the fluorescent signal activation of the bispecific antibody of the present invention in the co-incubation system of CHO-S cells overexpressing human PD-L1 and cells overexpressing human 4-1BB Jurkat NF-AT luciferase reporter gene.
Figure 5C shows the detection results of the fluorescent signal activation of the bispecific antibody of the present invention in the co-incubation system of CT-26 cells overexpressing human PD-L1 and cells overexpressing human 4-1BB Jurkat NF-AT luciferase reporter gene.
Figure 6A shows the detection results of the T cell activation of the bispecific antibody of the present invention in the co-incubation system of CHO-S cells and human primary T cells.
Figure 6B shows the detection results of the bispecific antibody of the present invention to activate T cells to secrete IL-2 in the co-incubation system of PD-L1 CHO-S cells and human primary T cells.
Figure 7 shows the detection results of the bispecific antibody of the present invention to activate T cells in a mixed lymphocyte reaction system.
Figure 8 shows the detection results of the half-life of the bispecific antibody of the present invention in mice.
Figure 9 shows the detection results of the pharmaceutical effect of the bispecific antibody of the present invention in the model of PD-L1/PD-1/4-1BB triple transgenic mice inoculated with human PD-L1 CT-26 cells.
Figure 10 shows the detection results of the pharmaceutical effect of the bispecific antibody of the present invention in the model of human PD-L1/4-1BB double transgenic mice inoculated with human PD-L1 MC38 cells.
Figure 11 shows the liver tissue HE staining results for the bispecific antibody of the present invention in the human 4-1BB mouse toxicity test. Each group in the horizontal direction shows the results of 4 mice, respectively.
Figure 12 shows the liver tissue CD8 IHC staining results for the bispecific antibody of the present invention in the human 4-1BB mouse toxicity test.

Some sequences involved in the present invention are shown in Table B below:

**Table B**

| Name or description of sequence | SEQ ID NO: | Specific composition of sequence |
|---|---|---|
| Peptide chain of bispecific antibody Bi-400 | 1 | QVQLVESGGGVVQPGRSLRLSCAASGSTFSIVAM GWYRQAPGKQRELVASIITGDGDTNYADSVKGR FTISRDNSKNTMYLQMNSLKPEDTAVYYCYART GYGSSWLMGHEYDYWGQGTQVTVSSTHTCPPCP APEAAGGPSVFLFPPKPKDTLMISRTPEVTCVVVD VSHEDPEVKFNWYVDGVEVHNAKTKPREEQYNS TYRVVSVLTVLHQDWLNGKEYKCKVSNKALPAP IEKTISKAKGQPREPQVYTLPPSREEMTKNQVSLT CLVKGFYPSDIAVEWESNGQPENNYKTTPPVLDS DGSFFLYSKLTVDKSRWQQGNVFSCSVMHEALH NHYTQKSLSLSPGGGGGSGGGGSGGGGSGGGGS EVQLQESGGGLVQPGGSLRLSCAASGFTFSSYWM YWLRQAPGKGLEWVSSINSDSSSTYYRDSVKGRF TISRDNAKNTLYLQMNSLKSEDTAVYYCAKDPG GYAKGQGTQVTVSS |
| Anti4-1BB nanobody HZ-L-Yr-13&14-16-01 | 2 | QVQLVESGGGVVQPGRSLRLSCAASGSTFSIVAM GWYRQAPGKQRELVASIITGDGDTNYADSVKGR FTISRDNSKNTMYLQMNSLKPEDTAVYYCYART GYGSSWLMGHEYDYWGQGTQVTVSS |
| IgG1 Fc (LALA) | 3 | THTCPPCPAPEAAGGPSVFLFPPKPKDTLMISRTP |
| | | EVTCVVVDVSHEDPEVKFNWYVDGVEVHNAKT KPREEQYNSTYRVVSVLTVLHQDWLNGKEYKCK VSNKALPAPIEKTISKAKGQPREPQVYTLPPSREE MTKNQVSLTCLVKGFYPSDIAVEWESNGQPENN YKTTPPVLDSDGSFFLYSKLTVDKSRWQQGNVFS CSVMHEALHNHYTQKSLSLSPG |
| Anti-PD-L1 nanobody C-Ye-18-5 | 4 | EVQLQESGGGLVQPGGSLRLSCAASGFTFSSYWM YWLRQAPGKGLEWVSSINSDSSSTYYRDSVKGRF TISRDNAKNTLYLQMNSLKSEDTAVYYCAKDPG GYAKGQGTQVTVSS |
| (G₄S)₄ linker | 5 | GGGGSGGGGSGGGGSGGGGS |
| Peptide chain of bispecific antibody Bi-088 | 6 | EVQLQESGGGLVQPGGSLRLSCAASGFTFSSYWM YWLRQAPGKGLEWVSSINSDSSSTYYRDSVKGRF TISRDNAKNTLYLQMNSLKSEDTAVYYCAKDPG GYAKGQGTQVTVSSTHTCPPCPAPEAAGGPSVFL FPPKPKDTLMISRTPEVTCVVVDVSHEDPEVKFN WYVDGVEVHNAKTKPREEQYNSTYRVVSVLTVL HQDWLNGKEYKCKVSNKALPAPIEKTISKAKGQP REPQVYTLPPSREEMTKNQVSLTCLVKGFYPSDIA VEWESNGQPENNYKTTPPVLDSDGSFFLYSKLTV DKSRWQQGNVFSCSVMHEALHNHYTQKSLSLSP GGGGGSGGGGSGGGGSGGGGSQVQLVESGGGV VQPGRSLRLSCAASGSTFSIVAMGWYRQAPGKQ RELVASIITGDGDTNYADSVKGRFTISRDNSKNT MYLQMNSLKPEDTAVYYCYARTGYGSSWLMGH EYDYWGQGTQVTVSS |
| Peptide chain #1 of bispecific antibody Bi-401-091 (i.e., peptide chain #1 bispecific antibody Bi-061-091) | 7 | EVQLQESGGGLVQPGGSLRLSCAASGFTFSSYWM YWLRQAPGKGLEWVSSINSDSSSTYYRDSVKGRF TISRDNAKNTLYLQMNSLKSEDTAVYYCAKDPG GYAKGQGTQVTVSSDKTHTCPPCPAPEAAGGPSV FLFPPKPKDTLMISRTPEVTCVVVDVSHEDPEVKF NWYVDGVEVHNAKTKPREEQYNSTYRVVSVLT VLHQDWLNGKEYKCKVSNKALPAPIEKTISKAK GQPREPQVCTLPPSRDELTKNQVSLSCAVKGFYP SDIAVEWESNGQPENNYKTTPPVLDSDGSFFLVS |
| | | KLTVDKSRWQQGNVFSCSVMHEALHNHYTQKSL SLSPGGGGGSGGGGSGGGGSGGGGSQVQLVESG GGVVQPGRSLRLSCAASGSTFSIVAMGWYRQAP GKQRELVASIITGDGDTNYADSVKGRFTISRDNSK NTMYLQMNSLKPEDTAVYYCYARTGYGSSWLM GHEYDYWGQGTQVTVSS |
| IgG1 Fc with hole mutation (LALA) | 8 | DKTHTCPPCPAPEAAGGPSVFLFPPKPKDTLMISR TPEVTCVVVDVSHEDPEVKFNWYVDGVEVHNA KTKPREEQYNSTYRVVSVLTVLHQDWLNGKEYK CKVSNKALPAPIEKTISKAKGQPREPQVCTLPPSR DELTKNQVSLSCAVKGFYPSDIAVEWESNGQPEN NYKTTPPVLDSDGSFFLVSKLTVDKSRWQQGNVF SCSVMHEALHNHYTQKSLSLSPG |
| Peptide chain #2 of bispecific antibody Bi-401-091, i.e., IgG1 Fc with knob mutation (LALA) | 9 | DKTHTCPPCPAPEAAGGPSVFLFPPKPKDTLMISR TPEVTCVVVDVSHEDPEVKFNWYVDGVEVHNA KTKPREEQYNSTYRVVSVLTVLHQDWLNGKEYK CKVSNKALPAPIEKTISKAKGQPREPQVYTLPPCR DELTKNQVSLWCLVKGFYPSDIAVEWESNGQPE NNYKTTPPVLDSDGSFFLYSKLTVDKSRWQQGN VFSCSVMHEALHNHYTQKSLSLSPG |
| Peptide chain #2 of bispecific antibody Bi-061-091 | 10 | EVQLQESGGGLVQPGGSLRLSCAASGFTFSSYWM YWLRQAPGKGLEWVSSINSDSSSTYYRDSVKGRF TISRDNAKNTLYLQMNSLKSEDTAVYYCAKDPG GYAKGQGTQVTVSSDKTHTCPPCPAPEAAGGPSV FLFPPKPKDTLMISRTPEVTCVVVDVSHEDPEVKF NWYVDGVEVHNAKTKPREEQYNSTYRVVSVLT VLHQDWLNGKEYKCKVSNKALPAPIEKTISKAK GQPREPQVYTLPPCRDELTKNQVSLWCLVKGFYP SDIAVEWESNGQPENNYKTTPPVLDSDGSFFLYS KLTVDKSRWQQGNVFSCSVMHEALHNHYTQKSL SLSPG |
| Peptide chain of control antibody INBRX-105-1B | 11 | EVQLLESGGGEVQPGGSLRLSCAASGGIFAIKPIS WYRQAPGKQREWVSTTTSSGATNYAESVKGRFT ISRDNAKNTLYLQMSSLRAEDTAVYYCNVFEYW GQGTLVTVKPGGSGGSEVQLLESGGGEVQPGGSL |
| | | RLSCAASGFSFSINAMGWYRQAPGKRREFVAAIE SGRNTVYAESVKGRFTISRDNAKNTVYLQMSSLR AEDTAVYYCGLLKGNRVVSPSVAYWGQGTLVT VKPGGGGDKTHTCPPCPAPGGPSVFLFPPKPKDT LMISRTPEVTCVVVDVSHEDPEVKFNWYVDGVE VHNAKTKPREEQYNSTYRVVSVLTVLHQDWLNG KEYKCKVSNKALPAPIEKTISKAKGQPREPQVYT LPPSRDELTKNQVSLTCLVKGFYPSDIAVEWESN GQPENNYKTTPPVLDSDGSFFLYSKLTVDKSRWQ QGNVFSCSVMHEALHNHYTQKSLSLSPGK |
| Heavy chain of Utomilumab | 12 | EVQLVQSGAEVKKPGESLRISCKGSGYSFSTYWIS WVRQMPGKGLEWMGKIYPGDSYTNYSPSFQGQ VTISADKSISTAYLQWSSLKASDTAMYYCARGYG IFDYWGQGTLVTVSSASTKGPSVFPLAPCSRSTSE STAALGCLVKDYFPEPVTVSWNSGALTSGVHTFP AVLQSSGLYSLSSVVTVPSSNFGTQTYTCNVDHK PSNTKVDKTVERKCCVECPPCPAPPVAGPSVFLFP PKPKDTLMISRTPEVTCVVVDVSHEDPEVQFNWY VDGVEVHNAKTKPREEQFNSTFRVVSVLTVVHQ DWLNGKEYKCKVSNKGLPAPIEKTISKTKGQPRE PQVYTLPPSREEMTKNQVSLTCLVKGFYPSDIAV EWESNGQPENNYKTTPPMLDSDGSFFLYSKLTVD KSRWQQGNVFSCSVMHEALHNHYTQKSLSLSPG K |
| Light chain of Utomilumab | 13 | SYELTQPPSVSVSPGQTASITCSGDNIGDQYAHW YQQKPGQSPVLVIYQDKNRPSGIPERFSGSNSGNT ATLTISGTQAMDEADYYCATYTGFGSLAVFGGG TKLTVLGQPKAAPSVTLFPPSSEELQANKATLVCL ISDFYPGAVTVAWKADSSPVKAGVETTTPSKQSN NKYAASSYLSLTPEQWKSHRSYSCQVTHEGSTVE KTVAPTECS |
| Heavy chain of Urelumab | 14 | QVQLQQWGAGLLKPSETLSLTCAVYGGSFSGYY WSWIRQSPEKGLEWIGEINHGGYVTYNPSLESRV TISVDTSKNQFSLKLSSVTAADTAVYYCARDYGP GNYDWYFDLWGRGTLVTVSSASTKGPSVFPLAP |
| | | CSRSTSESTAALGCLVKDYFPEPVTVSWNSGALT SGVHTFPAVLQSSGLYSLSSVVTVPSSSLGTKTYT CNVDHKPSNTKVDKRVESKYGPPCPPCPAPEFLG GPSVFLFPPKPKDTLMISRTPEVTCVVVDVSQEDP EVQFNWYVDGVEVHNAKTKPREEQFNSTYRVVS VLTVLHQDWLNGKEYKCKVSNKGLPSSIEKTISK AKGQPREPQVYTLPPSQEEMTKNQVSLTCLVKGF YPSDIAVEWESNGQPENNYKTTPPVLDSDGSFFL YSRLTVDKSRWQEGNVFSCSVMHEALHNHYTQK SLSLSLGK |
| Light chain of Urelumab | 15 | EIVLTQSPATLSLSPGERATLSCRASQSVSSYLAW YQQKPGQAPRLLIYDASNRATGIPARFSGSGSGTD FTLTISSLEPEDFAVYYCQQRSNWPPALTFCGGTK VEIKRTVAAPSVFIFPPSDEQLKSGTASVVCLLNN FYPREAKVQWKVDNALQSGNSQESVTEQDSKDS TYSLSSTLTLSKADYEKHKVYACEVTHQGLSSPV TKSFNRGEC |
| CDR1 of heavy chain variable region HZ-L-Yr-13&14-16-01 | 16 | SGSTFSIVA |
| CDR2 of heavy chain variable region HZ-L-Yr-13&14-16-01 | 17 | IITGDGDTN |
| CDR3 of heavy chain variable region HZ-L-Yr-13&14-16-01 | 18 | YARTGYGSSWLMGHEYDY |
| CDR1 of heavy chain variable region C-Ye-18-5 | 19 | GFTFSSYW |
| CDR2 of heavy chain variable region C-Ye-18-5 | 20 | INSDSSST |
| CDR3 of heavy chain variable region C-Ye-18-5 | 21 | AKDPGGYA |
| (G₄S)₄G linker | 22 | GGGGSGGGGSGGGGSGGGGSG |

### Specific Models for Carrying Out the present invention

The present invention is further illustrated in conjunction with specific examples below. It should be understood that these examples are only used to illustrate the present invention and are not intended to limit the scope of the present invention. The experimental methods without giving specific conditions in the following examples are usually carried out according to conventional conditions, such as the conditions described in Sambrook et al., Molecular cloning: Laboratory Manual (New York: Cold Spring Harbor Laboratory Press, 1989), or according to the conditions recommended by the manufacturer. Percentages and parts are by weight unless otherwise indicated.

If not specified, the C-terminals of HZ-L-Yr-13&14-16-01 and C-Ye-18-5 used in Example 2 to Example 12 were directly ligated to IgG1 Fc (LALA mutation).

### Example 1: Cloning and expression of bispecific antibodies

### 1.1 Structure of antibody constructs

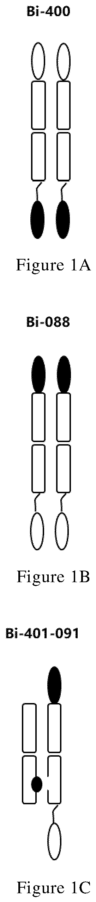

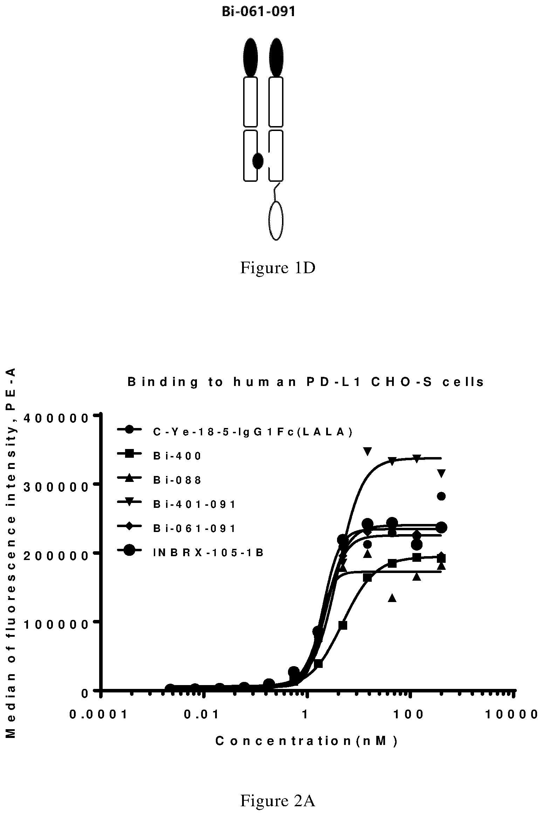

In this example, 4 anti-4-1BB/PD-L1 bispecific antibodies were constructed, namely:
Bi-400: It was composed of 2 identical polypeptide chains (the two peptide chains were linked by 2 pairs of disulfide bonds therebetween), and its structural diagram was shown in Figure 1A. The peptide chains had the amino acid sequence as set forth in SEQ ID NO: 1, which comprised the amino acid sequence of the anti-4-1BB nanobody HZ-L-Yr-13&14-16-01 (SEQ ID NO: 2), the C-terminal of the anti-4-1BB nanobody amino acid sequence was directly ligated to the human IgG1 Fc amino acid sequence (introduced with LALA mutation, SEQ ID NO: 3, named as IgG1 Fc (LALA)), and the N-terminal of anti-PD-L1 nanobody C-Ye-18-5 (publication number: CN 112480253A) (SEQ ID NO: 4) was ligated through a linker of 21 amino acid residues (G₄S)₄ (SEQ ID NO: 5) to the C-terminal of IgG1 Fc (LALA).
Bi-088: It was composed of 2 identical polypeptide chains, its structural schematic diagram was shown in Figure 1B, the peptide chains had an amino acid sequence as set forth in SEQ ID NO: 6, which comprised the anti-PD-L1 nanobody C-Ye-18 -5 (publication number: CN 112480253A) (SEQ ID NO: 4), the C-terminal of the nanobody amino acid sequence was ligated directly to the human IgG1 Fc amino acid sequence (introduced with LALA mutation, SEQ ID NO: 3), and the N-terminal of the anti-4-1BB nanobody HZ-L-Yr-13&14-16-01 amino acid sequence (SEQ ID NO: 2) is ligated through a linker of 21 amino acid residues (G₄S)₄ (SEQ ID NO: 5) to the C-terminal of IgG1 Fc (LALA).
Bi-401-091: It was composed of 2 polypeptide chains, and their structural diagrams were shown in Figure 1C, in which the peptide chain #1 had an amino acid sequence as set forth in SEQ ID NO: 7, which comprised the anti-PD-L1 nanobody C-Ye -18-5 (publication number: CN 112480253A) (SEQ ID NO: 4), the C-terminal of the nanobody amino acid sequence was ligated directly to the human IgG1 Fc amino acid sequence (introduced with LALA mutation, introduced with Knob-in-hole mutation, SEQ ID NO: 8), the N-terminal of the anti-4-1BB nanobody HZ-L-Yr-13&14-16-01 (SEQ ID NO: 2) was ligated through a linker of 21 amino acid residues (G₄S)₄ (SEQ ID NO: 5) to the C-terminal of the Fc; the peptide chain #2 had an amino acid sequence as set forth in SEQ ID NO: 9, which was the human IgG1 Fc amino acid sequence (introduced with LALA mutation, introduced with Knob-in-hole mutation, SEQ ID NO: 9).
Bi-061-091: It was composed of 2 polypeptide chains, and their structural diagrams were shown in Figure 1D, in which the peptide chain #1 had an amino acid sequence as set forth in SEQ ID NO: 7; the peptide chain #2 had an amino acid sequence as set forth in SEQ ID NO: 10, which comprised the anti-PD-L1 nanobody C-Ye-18-5 (publication number: CN 112480253A) (SEQ ID NO: 4), and the C-terminal of the nanobody amino acid sequence is ligated directly to human IgG1 Fc amino acid sequence (introduced with LALA mutation to reduce Fc function, introduced with Knob-in-Hole mutation to form heterodimer, SEQ ID NO: 9).

### 1.2 Gene cloning and protein preparation

Referring to the sequences in Table B, the gene fragments encoding the corresponding amino acid sequences were constructed into the pCDNA3.1 vector. For the peptide chain #1 and peptide chain #2 in Bi-401-091 and Bi-061-091, these two sequences were expressed in two different plasmids during transient transfection, and automatically formed disulfide bonds in the process of cell expression.

Using the ExpiCHO^{™} Expression System Kit (purchased from Thermo), the plasmids were transfected into Expi-CHO cells. The transfection method was carried out according to the manufacturer's instructions. After the cells were cultured for 5 days, the supernatant was collected and subjected to separation method using protein A magnetic beads (purchased from GenScript) to purify the target protein. The magnetic beads were resuspended with an appropriate volume of binding buffer (PBS+0.1% Tween 20, pH 7.4) (1-4 times the volume of magnetic beads), then added to the sample to be purified, and incubated at room temperature for 1 hour with gentle shaking. The sample was placed on a magnetic stand (purchased from Beaver), the supernatant was discarded, and the magnetic beads were washed 3 times with binding buffer. The elution buffer (0.1M sodium citrate, pH 3.2) was added according to 3-5 times the volume of the magnetic beads, shaken at room temperature for 5-10 minutes, and placed on the magnetic stand, the elution buffer was collected, and transferred and mixed well in a collection tube in which the neutralization buffer solution (1 M Tris, pH8.54) had been added. The prepared bispecific antibody samples were used in subsequent experiments.

### Example 2: Cell-based binding of bispecific antibodies

CHO cells overexpressing human or cynomolgus 4-1BB (PD-L1) were generated by transfection of pCHO1.0 vector (purchased from Invitrogen) encoding human or cynomolgus monkey 4-1BB (PD-L1) cDNA cloned into MCS (Multiple Cloning Site) (theoretically, this method could obtain the overexpression cells by transfection and stressed screening, and they would be confirmed by flow cytometry later). The overexpression cells were subjected to expansion culture and adjusted to have a cell density of 2×10⁶ cells/ml, then added to a 96-well flow plate, 100 µl/well, and centrifuged for later use. The purified 4-1BB antibody was diluted with PBS by 3-fold dilution starting from 400 nM to obtain a total of 12 points. The above-mentioned diluted sample was added to the above-mentioned 96-well flow plate with the cells, 100 µl/well, and incubated at 4°C for 30 minutes, then washing was performed twice with PBS. Goat F(ab')₂ anti-human IgG-Fc (PE) (purchased from Abcam) diluted 100 times with PBS was added, 100 µl/well, and incubated at 4°C for 30 minutes, then washing was performed twice with PBS. PBS was added to resuspend the cells, 100 µl/well, the detection was performed on CytoFlex (Bechman) flow cytometer and the corresponding MFI was calculated. INBRX-105-1B (there was only one peptide chain, the amino acid sequence was set forth in SEQ ID NO: 11. Referred to WO2017123650A2) was used as a bispecific antibody positive control (anti-PD-L1/4-1BB bispecific antibody from Inhibrx); the nanobody HZ-L-Yr-13&14-16-01 was ligated to human IgG1 Fc (LALA mutation) (SEQ ID NO: 3) as an anti-4-1BB positive control; the nanobody C-Ye-18-5 (SEQ ID NO: 5) was ligated to human IgG1 Fc (LALA mutation) (SEQ ID NO: 3) as an anti-PD-L1 positive control. In addition, two anti-4-1BB monoclonal antibodiesUtomilumab (patent number: US20120237498) (its heavy chain and light chain sequences were set forth in SEQ ID NO: 12 and SEQ ID NO: 13, respectively) and Urelumab (International Nonproprietary Names for Pharmaceutical Substances: 104, its heavy chain and light chain sequences were set forth in SEQ ID NO: 14 and SEQ ID NO: 15, respectively) were used as anti-4-1BB positive controls.

The experimental results were shown in Figure 2A to Figure 2E.

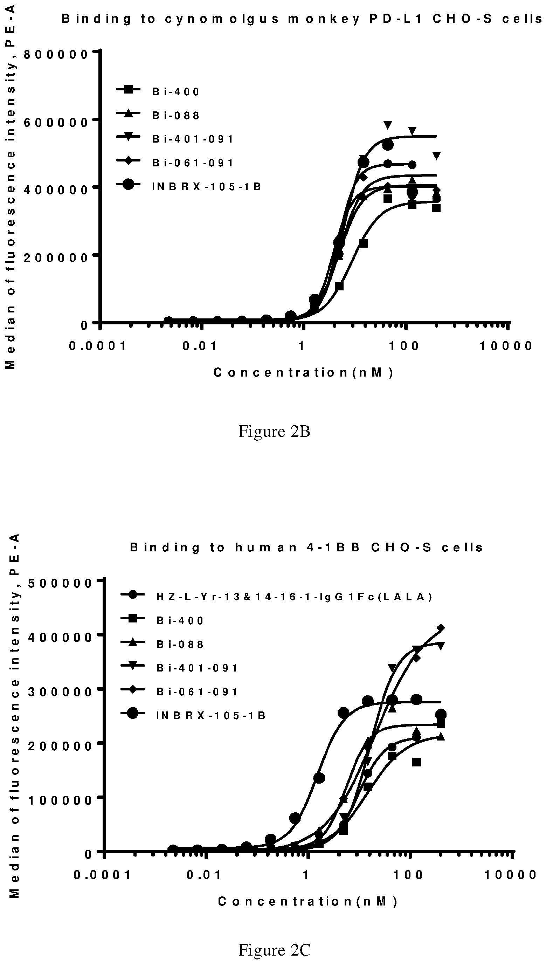

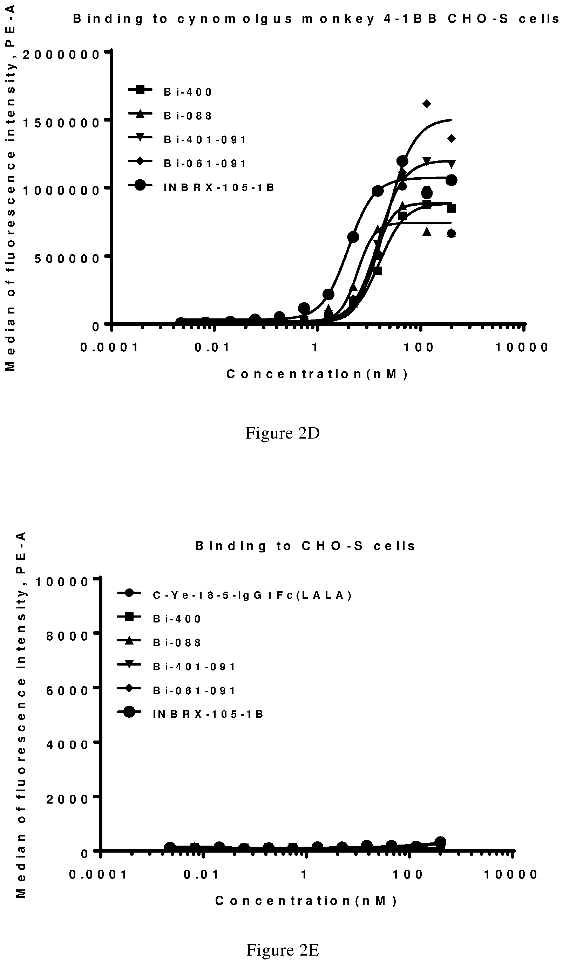

As shown in Figure 2A, the bispecific antibodies with different structures showed different binding activities to the human PD-L1-overexpressing CHO cells, and the binding activities of some bispecific antibodies were comparable to those of the control antibodies; as shown in Figure 2B, the bispecific antibodies with different structures showed different binding activities to the cynomolgus monkey PD-L1-overexpressing CHO cells, and the binding activities of some bispecific antibodies were comparable to those of the control antibodies; as shown in Figure 2C, the bispecific antibodies with different structures showed different binding activities to the human 4-1BB-overexpressing CHO cells, and the binding activities of some bispecific antibodies were comparable to those of the control antibodies; as shown in Figure 2D, the bispecific antibodies with different structures showed different binding activities to the cynomolgus monkey 4-1BB overexpressing CHO cells, and the binding activities of some bispecific antibodies were comparable to those of the control antibodies; as shown in Figure 2E, the bispecific antibodies with different structures showed no obvious non-specific binding to the CHO-S cells.

### Example 3: Bispecific antibodies blocking the binding of PD-L1 to PD-1 CHO cells

In this example, the expansion cultured CHO-hPD-1 cells (overexpressing human PD-1) were adjusted to a cell density of 2×10⁶ cells/ml, added to a 96-well flow plate, 100 µl/well, and centrifuged for later use. The purified bispecific antibody was diluted with PBS by 3-fold dilution starting from 400 nM to obtain a total of 12 points, the diluted sample was added to a 96-well sample dilution plate, 60 µl/well. Biotin-labeled human PD-L1 protein (purchased from AcroBiosystems) was added at 60 µl/well at the same time to achieve a final concentration of 500 ng/ml, and incubated at 4°C for 30 minutes. The co-incubation sample was added at 100 µl/well into the above-mentioned 96-well flow plate with cells, incubated at 4°C for 30 minutes, and washed twice with PBS. APC goat anti-mouse IgG antibody (purchased from Biolegend) diluted 100 times with PBS was added at 100 µl/well, incubated at 4°C for 30 minutes, and washed twice with PBS. PBS was added at 100 µl/well to resuspend the cells, the detection was performed on CytoFlex (Bechman) flow cytometer and the corresponding MFI was calculated.

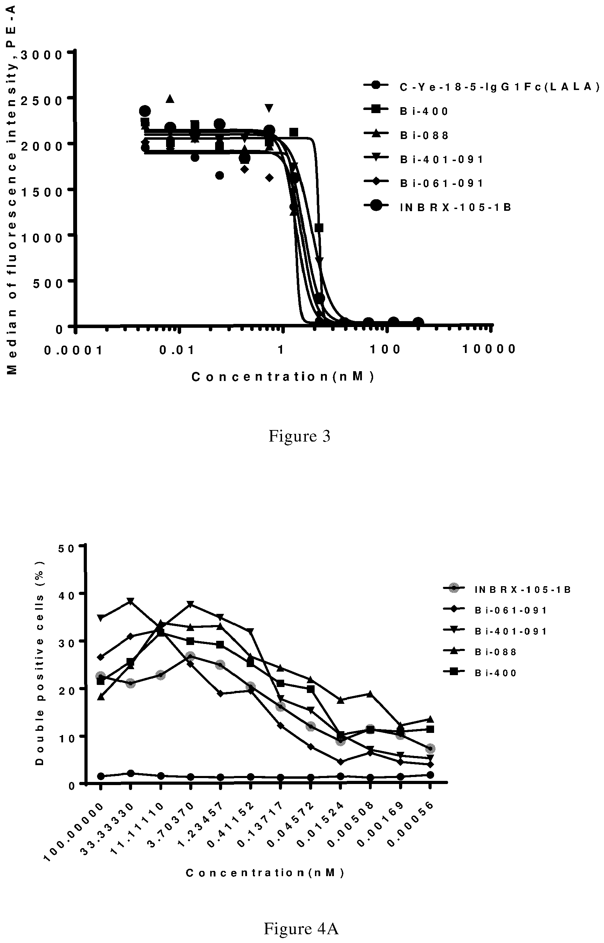

The results were shown in Figure 3. The bispecific antibodies with different structures of the present invention all could block the binding of PD-L1 to PD-1, and the blocking level varied with the structure, in which the blocking activity of the Bi-088 molecule was comparable or even superior to those of the control antibodies.

### Example 4: Detection of bispecific antibody cell based co-binding

In this example, CHO cells overexpressing human 4-1BB were labeled with CFSE (purchased from Thermo), and CHO cells overexpressing human PD-L1 were labeled with CTV (purchased from Thermo). The cells were resuspended in PBS and adjusted to have a cell density of 4×10⁶ cells/ml, respectively, the two cell suspensions were mixed in equal proportions, added at 50 µl/well to a 96-well U-bottom plate (purchased from Thermo), and added at the same time with the bispecific antibody or control antibody at 50 µl/well that was gradiently diluted with PBS by 3-fold dilution starting from the final concentration of 100nM to obtain a total of 12 points, incubated at 37°C for two hours, the detection was performed on CytoFlex (Bechman) flow cytometer and the proportion of double-positive cells was calculated.

The results were shown in Figure 4A. The bispecific antibody could simultaneously bind to the human 4-1BB overexpressed CHO cells and the human PD-L1 overexpressed CHO cells, and the binding activity varied with the structure of the bispecific antibody, in which the binding activities of Bi-088, Bi-400 and Bi-401-091 molecules were comparable to or even better than those of the control antibody.

### Example 5: Bispecific antibody protein based co-binding assay

Human 4-1BB protein (purchased from ACRO) was dissolved according to the instruction, diluted to 1 µg/ml with ELISA coating solution (purchased from Shanghai Sangon), coated on ELISA plate at 100 µl/well, allowed to stand overnight at 4°C, washed 3 times with PBST, 5% BSA (purchased from Shanghai, Sangon) was added at 200 µl/well to perform blocking at room temperature for 1 hour. The blocking solution was discarded, the bispecific antibody or control antibody was serially diluted with 1% BSA by 3-fold dilution starting from the final concentration of 100 nM to obtain a total of 12 points and added at 100 µl/well, and incubated at room temperature for 2 hours. Washing was performed with PBST 3 times, biotin-labeled PD-L1 protein (purchased from Kactus) diluted with 1% BSA was added at 100 µl/well, and the diluted protein sample had a concentration of 1 ug/ml, and incubation was carried out at room temperature for 1 hour. Washing was performed 3 times with PBST, SA-HRP (purchased from abeam) diluted with 1% BSA (1:10000) was added at 100 µl/well, and incubated at room temperature for 0.5 hours. Washing was performed 3 times with PBST, ELISA chromogenic solution (purchased from Solarbio) was added at 100 µl/well and reacted for 3 minutes at room temperature, ELISA stop solution (purchased from Solarbio) was added at 50 µl/well, and the absorbance value at 450 nm was read.

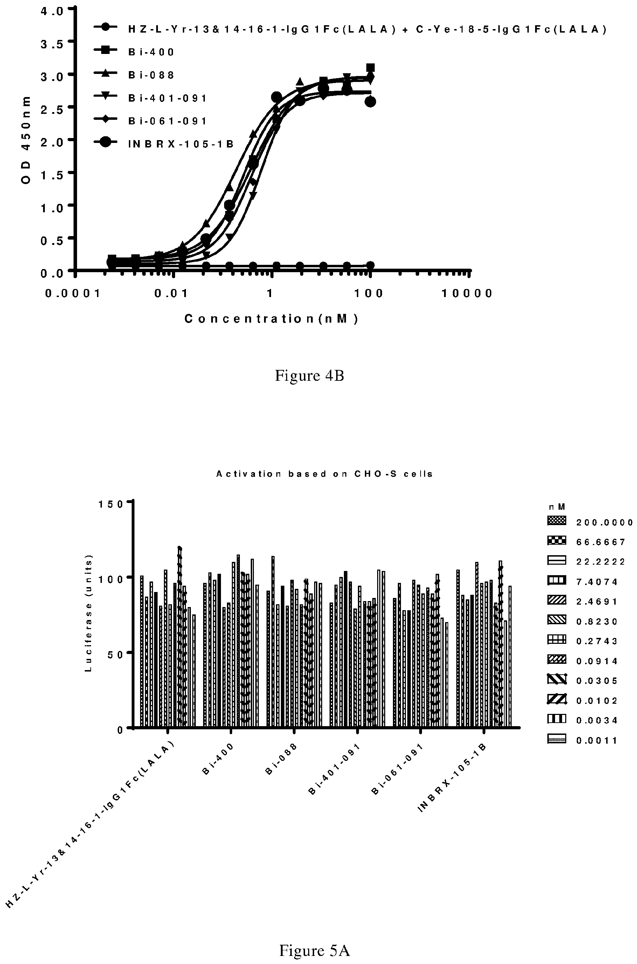

The results were shown in Figure 4B. The bispecific antibody could simultaneously bind to human 4-1BB and human PD-L1 proteins, and the binding activity varied with the molecular structure. Among them, the binding activity of Bi-088 molecule was comparable to or even better than that of the control antibody.

### Example 6: Detection of bispecific antibody activity (luciferase reporter cell based assay)

The plasmid encoding human 4-1BB and the plasmid encoding NF-κB luciferase reporter gene (purchased from Promega) were co-transfected into Jurkat cells to obtain hu4-1BB Jurkat-NF-κB cells. The hu4-1BB Jurkat-NF-κB cells were subjected to expansion culture, the cells were resuspended in 1640 complete medium to 4×10⁶ cells/ml. CHO-S cells overexpressing human PD-L1, CHO-S cells, and CT-26 cells overexpressing human PD-L1 were diluted with 1640 complete medium to 4×10⁵ cells/ml. The above three kinds of cell suspensions were separately mixed with hu4-1BB Jurkat-NF-κB cells at a ratio of 1:1, added at 50 µl/well into a sterile 96-well white bottom plate (purchased from Nunc), and added with the bispecific antibody sample serially diluted with 1640 complete medium by 3-fold dilution starting from the final sample concentration of 200 nM to obtain a total of 12 points. Incubation was performed at 37°C, 5% CO₂ for 16 hours, and the luciferase signal was detected.

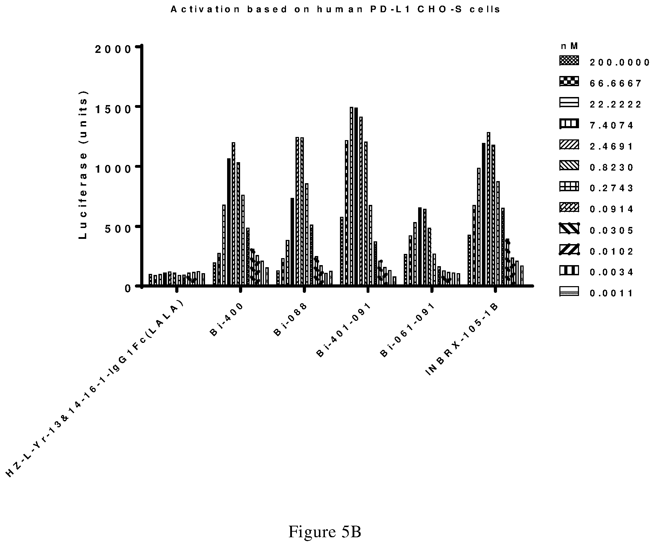

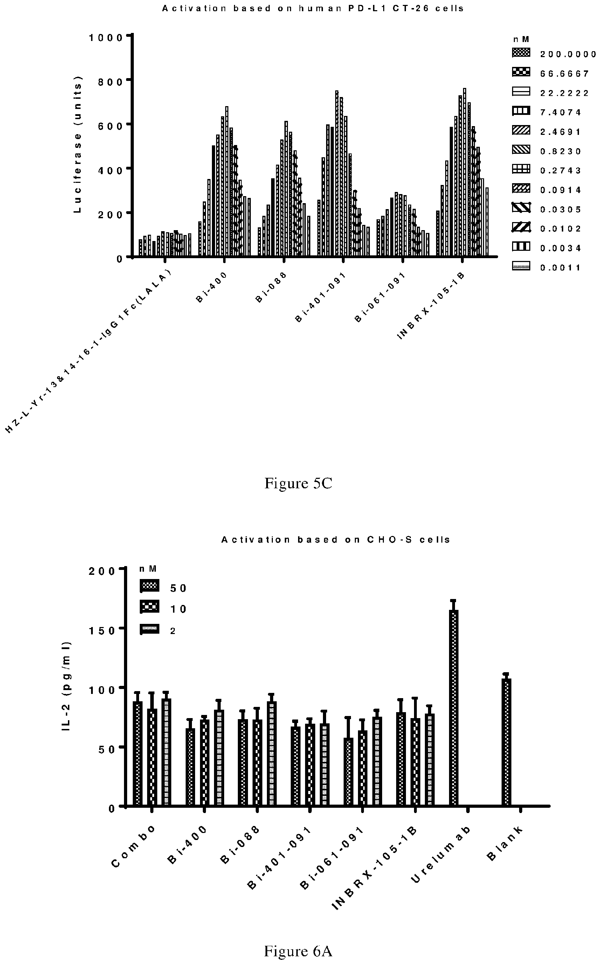

The results were shown in Figures 5A, 5B and 5C. No obvious signal activation was observed in the bispecific antibody samples in the co-incubation system of hu4-1BB Jurkat-NF-κB cells and CHO-S cells. Significant signal activation activity was observed in the co-incubation system of hu4-1BB Jurkat-NF-κB cells and CHO-S cells overexpressing human PD-L1 or CT-26 cells overexpressing human PD-L1. This was due to the crosslinking effect caused by PD-L1, which made the 4-1BB on Jurkat cells bound to the other end of the antibody clustered together, thereby activated the downstream NF-κB pathway. It suggests that in the human body, the bispecific antibodies can activate human T cell's NF-κB pathway under the PD-L1 crosslinking effect, further activate T cells, and kill tumors. Among them, the activation activities of Bi-088, Bi-400, and Bi-401-091 were comparable to or even better than that of the control antibody.

### Example 7: Detection of bispecific antibody activity (primary T cells activation assay)

CHO-S overexpressing human PD-L1 or CHO cells were treated with mitomycin for 4 hours, and adjusted with X-VIVO15 medium to have a cell density of 2×10⁶ cells/ml. OKT-3 antibody (purchased from Biolegend) was diluted to 1 µg/ml with sterile PBS (purchased from Hyclone), coated on a 96-well cell culture flat bottom plate (purchased from Thermo) at 100 µl/well, and incubated at 37°C for two hours. Frozen human PBMCs (purchased from Shanghai Saili) were resuscitated, isolated with human T cell isolation and purification kit (purchased from Stemcell) to obtain T cells, the T cells were resuspended with X-VIVO15 medium (purchased from LONZA), and adjusted to have a cell density of 2×10⁶ cells/ml, and mixed with the mitomycin-treated CHO-S cells or CHO cells overexpressing human PD-L1 in equal proportion. After the antibody coating was completed, the coating solution was discarded, washing was performed twice with PBS, the PBS was discarded, the above cell mixture solution was added at 100 µl/well, and at the same time the bispecific antibody sample serially diluted with X-VIVO15 was added at 100 µl/well, the sample final concentration or the combo group was added with HZ-L-Yr-13&14-16-1-IgG1Fc(LALA) + C-Ye-18-5-IgG1Fc(LALA), the final concentration was 50, 10, 2 nM. Incubation was performed at 37°C, 5% CO₂ for 3 days, and the supernatant was collected to detect the IL-2 content.

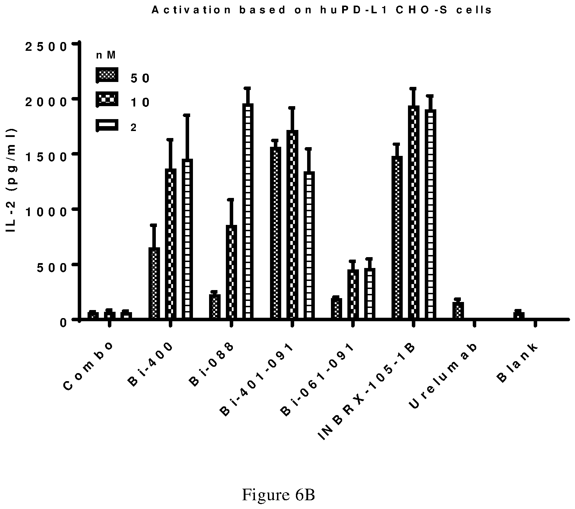

The experimental results were shown in Figures 6A and 6B. There was no obvious signal activation for the bispecific antibody sample in the co-incubation system of T cells and CHO-S cells, but there were significant T cell activation and IL-2 release in the co-incubation system of T cells and CHO-S cells overexpressing human PD-L1, in which, the activity of Bi-088, Bi-400, and Bi-401-091 molecules were comparable to or even better than those of the control antibody.

### Example 8: Activity of bispecific antibody in mixing lymphocyte reaction assay

PBMCs (purchased from SAILY BIO, SLB-HPB) were resuscitated, centrifuged, the PBMCs were resuspended with 10 ml of X-VIVO-15 medium (purchased from LONZA), cultured in a cell culture incubator at 37°C for 2 hours, and the adherent cells were removed by sucking. 10 ml of DC medium was added: 10 ng/ml GM-CSF (purchased from R&D) and 20 ng/ml IL-4 (purchased from R&D) were added to X-VIVO-15 medium, and cultured for 3 days, 5 ml of DC medium was supplemented, the culturing was continued to the 6^{th} day, DC maturation medium was added, and cultured for 2 days, wherein the DC maturation medium was prepared by adding 1000 U/ml TNF-α (purchased from R&D), 10 ng/ml IL-6 (purchased from R&D), 5 ng/ml IL-1β (purchased from R&D), and 1 µM PGE2 (purchased from Tocris) to X-VIVO-15 medium. The mature DC cells were collected, and adjusted with X-VIVO-15 medium to have a cell density to 2×10⁵ cells/ml.

PBMCs from another donor (purchased from SAILY BIO, SLB-HPB) were resuscitated, centrifuged, and the PBMCs were resuspended with 10 ml of X-VIVO-15 medium. The T cells were enriched with T cell sorting kit (purchased from Stemcell), the T cells were resuspended in X-VIVO-15, adjusted to have a cell density to 2×10⁶ cells/ml, and mixed with the mature DC cells collected above at a ratio of 1:1, and added at 100 µl/well to a 96-well U-bottom plate. At the same time, the bispecific antibody sample diluted with X-VIVO-15 medium was added at 100 µl/well, which had the final concentration of 10, 2, 0.4, 0.08, 0.016 nM, culturing was performed for 3 days, the supernatant was collected, and IL-2 expression level was detected by ELISA (purchased from eBioscience).

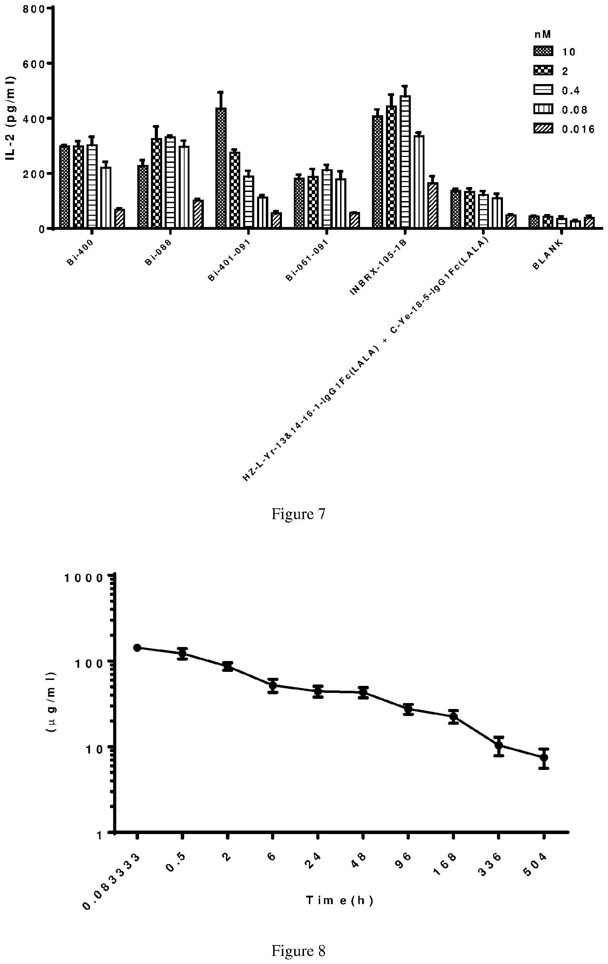

The experimental results were shown in Figure 7. The bispecific antibody samples could activate T cells to release IL-2 in the mixed lymphocyte reaction system, and the activation levels of Bi-088 and Bi-400 were comparable to that of the control antibody.

### Example 9: Half-life of bispecific antibodies in mice

Balb/c mice were used in the experiment, half male and half female, 6 for each blood collection point, 12/12 hour light/dark adjustment, temperature of 24±2°C, humidity of 40% to 70%, free access to water and diet. On the day of the experiment, the bispecific antibody molecule Bi-088 was injected into the tail vein of Balb/c mice once, and the injection dose was 10 mg/kg.

Blood collection time points: 5 minutes, 0.5 hours, 2 hours, 6 hours, 24 hours, 48 hours, 96 hours, 168 hours, 336 hours, and 504 hours after administration, blood was collected from the mouse orbit. The whole blood samples were placed at 2-8°C for 30 minutes, centrifuged at 12,000rpm for 5 minutes to collect sera, then the sera were centrifuged at 2-8°C, 12,000rpm for 5 minutes, stored at -80°C, the contents of bispecific antibody molecules in the sera were detected by ELISA, and the average value thereof was calculated. The results were shown in Figure 8, and the half-life of the bispecific antibody molecule of the present invention in mice was about 174 hours.

### Example 10: Pharmaceutical effect of bispecific antibody in mice

In this experiment, mouse colorectal cancer CT-26 cells expressing human PD-L1 (h-PD-L1 KI CT-26) were transplanted in human 4-1BB/PD-L1/PD-1 transgenic mice (purchased from GemPharmatech) to determine the anti-tumor effect of bispecific antibodies. First, h-PD-L1 KI CT-26 tumor-bearing mouse model was established by subcutaneous inoculation, each mouse was inoculated with 0.6×10⁶ cells, the grouping was carried out when the average tumor volume reached 100-200 mm³, 6 mice in each group. Treatment was performed by intraperitoneal injection with different antibodies at different doses, and the changes in tumor volume and body weight of mice in each group were monitored at a frequency of once per 2-3 days, and the monitoring was continued for 2 to 3 weeks. The administration doses and models are shown in Table 1.

**Table 1: Experimental protocol for tumor suppressive activity of bispecific antibodies**

| Group | Dosage of administration | Times of administration |
|---|---|---|
| Negative control | N/A | Once every two days, 3 times |
| Bi-088 | 4 mg/kg | Once every two days, 3 times |
| Bi-401-091 | 3 mg/kg | Once every two days, 3 times |
| INBRX-105-1B | 4 mg/kg | Once every two days, 3 times |
| C-Ye-18-5 + HZ-L-Yr-13&14-16-01 | 3 mg/kg+3 mg/kg | Once every two days, 3 times |

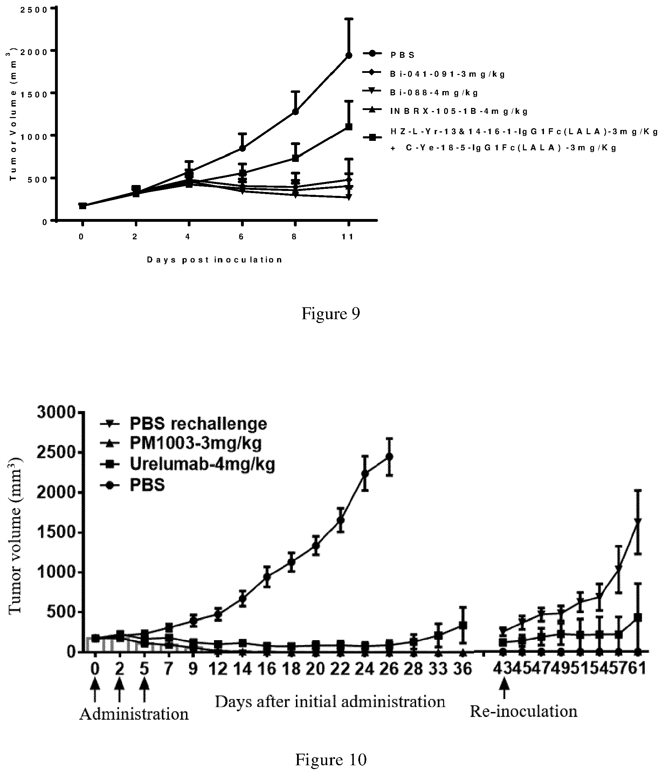

The results were shown in Figure 9.

The results showed that the bispecific antibody could significantly inhibit the growth of h-PD-L1 KI CT-26 cells, and the inhibitory activity was significantly better than that of the combination drug group, and the inhibitory activity of Bi-088 was comparable to or better than that of the control antibody INBRX-105-1B.

### Example 11: Pharmacodynamic model of human PD-L1/4-1BB double transgenic mice transplanted with huPD-L1 MC-38 cells

In this experiment, MC-38 cells expressing human PD-L1 (huPD-L1 MC-38) were used to determine the anti-tumor effect of Bi-088 in human PD-L1/4-1BB transgenic mice (purchased from Biocytogen Biotechnology Co., Ltd.). Firstly, the huPD-L1 MC-38 tumor-bearing mouse model was established by subcutaneous inoculation, each mouse was inoculated with 1×10⁶ cells, and grouping was performed when the average tumor volume reached 80-120mm³. Six mice in each group were treated with different antibodies at different doses by intraperitoneal injection, and the doses and methods of administration were shown in Table 2 below. After the mouse tumors completely regressed, a new round of huPD-L1 MC-38 cells were inoculated on the contralateral side of the mice, and the changes in tumor volume and body weight of the mice in each group were monitored. The monitoring frequency was once per 2-3 days, a total of 8 weeks.

**Table 2: Experimental protocol for tumor suppressive activity of Bi-088**

| Group | Dosage of administration | Time of administration |
|---|---|---|
| Negative control | N/A | day 0, 2,5 |
| Urelumab | 4 mg/kg | day 0, 2,5 |
| Bi-088 | 3 mg/kg | day 0, 2,5 |
| INBRX-105-1B | 3 mg/kg | day 0, 2,5 |

The results were shown in Table 3 and Figure 10.

**Table 3: Tumor regression in mice after reinoculation**

| Group/Number of mice with complete tumor regression | PBS | Urelumab | Bi-088 | INBRX-105-1B |
|---|---|---|---|---|
| Initial treatment | 0/6 | 4/6 | 6/6 | 6/6 |
| Reinoculation | 0/6 | 2/6 | 6/6 | 6/6 |

The results showed that after inoculation of huPD-L1 MC-38 cells, the tumor volume of the negative control group continued to increase, while the tumors of the 6 mice in the Bi-088 group completely regressed after treatment, and 31 days after the last administration, even huPD-L1 MC-38 cells were inoculated again on the contralateral side, the tumor cells did not grow as well.

### Example 12: Hepatotoxicity test in human 4-1BB Transgenic mice

In this experiment, the hepatotoxicity of Bi-088 was determined in human 4-1BB transgenic mice (purchased from Biocytogen Biotechnology Co., Ltd.), 4 mice in each group. The Bi-088 molecule and the control molecule were intraperitoneally administered in the manner shown in Table 4 below. On the 20^{th} day after the initial administration, the mice were euthanized and the liver tissues were taken for embedding and fixing, sectioning, HE staining and CD8 positive cell staining.

**Table 4: Protocol for hepatotoxicity test in 4-1BB transgenic mice**

| Group | Dosage of administration | Times of administration |
|---|---|---|
| Negative control | N/A | Once per 5 days, ×4 times |
| Urelumab | 10 mg/kg | Once per 5 days, ×4 times |
| Bi-088 | 7.5 mg/kg | Once per 5 days, ×4 times |
| INBRX-105-1B | 7.5 mg/kg | Once per 5 days, ×4 times |

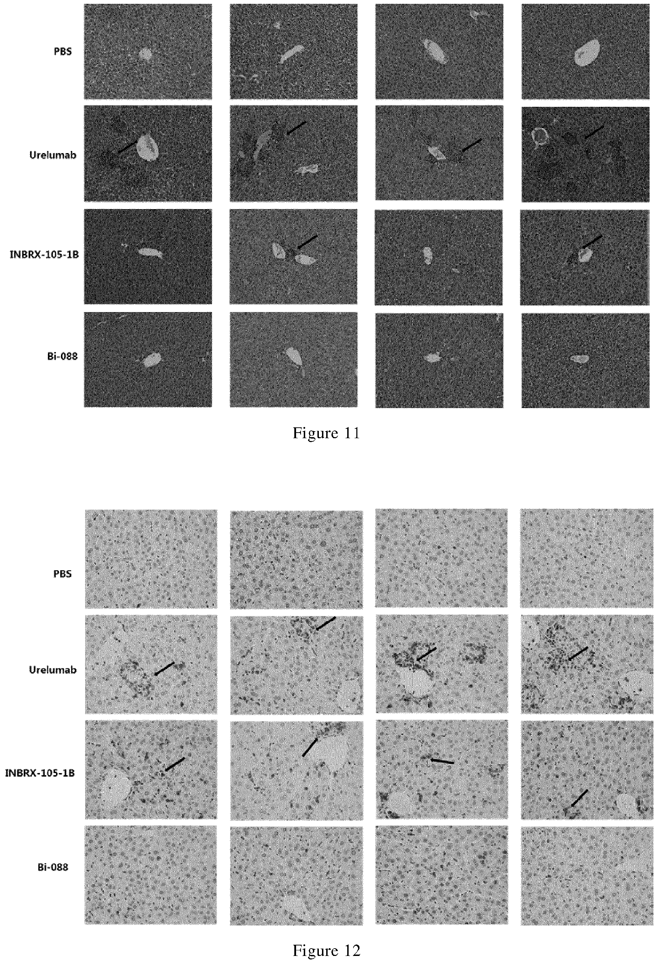

The results were shown in Figure 11 and Figure 12.

The results showed that the mice in the Urelumab group had obvious mononuclear cell infiltration and positive staining of CD8 cells in the liver, some mice in the INBRX-105-1B group had obvious mononuclear cell infiltration and positive staining of CD8 cells in the liver, and the mice in the Bi-088 group showed no obvious mononuclear cell infiltration and positive staining of CD8 cells in the liver in mice.

Although specific embodiments of the present invention have been described in detail, those skilled in the art will understand that based on all the teachings that have been disclosed, various modifications and substitutions can be made to those details, and these changes are all within the scope of the present invention. The full scope of the present invention is given by the appended claims and any equivalents thereof.

## Claims

1. A bispecific antibody, which comprises:
a first protein functional region targeting 4-1BB, and
a second protein functional region targeting PD-L1 or PD-1;
wherein:
the first protein functional region is an anti-4-1BB single-domain antibody;
the second protein functional region is an anti-PD-L1 antibody, an anti-PD-1 antibody, an anti-CTLA-4 antibody or an anti-HER-2 antibody, or an antigen-binding fragment thereof.

2. The bispecific antibody according to claim 1, wherein the anti-4-1BB single-domain antibody comprises a heavy chain variable region, and the heavy chain variable region comprises CDR1 having an amino acid sequence as set forth in SEQ ID NO: 16, CDR2 with an amino acid sequence as set forth in SEQ ID NO: 17, and CDR3 with an amino acid sequence as set forth in SEQ ID NO: 18;
preferably, the anti-4-1BB single-domain antibody has an amino acid sequence as set forth in SEQ ID NO: 2.

3. The bispecific antibody according to any one of claims 1 to 2, wherein the second protein functional region is an anti-PD-L1 single-domain antibody, an anti-PD-1 single-domain antibody, an anti-CTLA-4 single-domain antibody or an anti-HER-2 single-domain antibody;
preferably, the anti-PD-L1 single-domain antibody comprises a heavy chain variable region, and the heavy chain variable region comprises CDR1 with an amino acid sequence as set forth in SEQ ID NO: 19, CDR2 with an amino acid sequence as set forth in SEQ ID NO: 20, and CDR3 with an amino acid sequence as set forth in SEQ ID NO: 21;
preferably, the anti-PD-L1 single-domain antibody has an amino acid sequence as set forth in SEQ ID NO: 4.

4. The bispecific antibody according to any one of claims 1 to 3, wherein the bispecific antibody further comprises one or more (e.g., 2 or 3) Fc fragments of IgG, such as Fc fragment of IgG1, IgG2, IgG3 or IgG4;
or, the bispecific antibody further comprises one or more (e.g., 2 or 3) constant regions of IgG, such as heavy chain constant region of IgG1, IgG2, IgG3 or IgG4;
preferably, the Fc fragment of IgG or constant region of IgG is located between the first protein functional region and the second protein functional region.

5. The bispecific antibody according to claim 4, wherein the Fc fragment of IgG or the heavy chain constant region of IgG is ligated to the C-terminal of the first protein functional region, and/or is ligated to the C-terminal of the second protein functional region;
the Fc fragment of IgG or the heavy chain constant region of IgG is ligated directly or through a linker to the first protein functional region; and the Fc fragment of IgG or the heavy chain constant region of IgG is ligated directly or through a linker to the second protein functional region;
preferably, the linker has an amino acid sequence independently selected from SEQ ID NO: 5 and SEQ ID NO: 22.

6. The bispecific antibody according to any one of claims 4 to 5, wherein, according to the EU numbering system, the Fc fragment of IgG or the heavy chain constant region of IgG comprises a L234A mutation and a L235A mutation; optionally, the Fc fragment of IgG further comprises a G237A mutation;
preferably, the Fc fragment of IgG is an Fc fragment of IgG1 comprising a L234A mutation and a L235A mutation; preferably, the Fc fragment of IgG1 has an amino acid sequence as set forth in SEQ ID NO: 3;
preferably, the Fc fragment of IgG further comprises a Knob-in-hole mutation;
preferably, the Fc fragment of IgG1 further comprises a Knob-in-hole mutation; preferably, the Fc fragment of IgG1 has an amino acid sequence as set forth in SEQ ID NO: 8 or SEQ ID NO: 9.

7. The bispecific antibody according to any one of claims 1 to 6, wherein the bispecific antibody comprises the following first peptide chain:
the first peptide chain sequentially comprises the first protein functional region, the Fc fragment of IgG or the heavy chain constant region of IgG, a linker and the second protein functional region from the N-terminal to the C-terminal; or comprises the second protein functional region, the Fc fragment of IgG or the heavy chain constant region of IgG, a linker and the first protein functional region;
preferably, the first peptide chain has an amino acid sequence as set forth in SEQ ID NO: 1 or SEQ ID NO: 6.

8. The bispecific antibody according to claim 7, wherein the bispecific antibody is a dimer formed by two first peptide chains, preferably a dimer formed by the first peptide chains as set forth in SEQ ID NO: 1 and/or SEQ ID NO: 6;
preferably, the two first peptide chains are linked by 2 or 3 pairs of disulfide bonds; Optionally, the two first peptide chains further comprise a Knob-in-hole mutation.

9. The bispecific antibody according to claim 7, wherein the bispecific antibody further comprises the following second peptide chain:
the second peptide chain comprises the Fc fragment of IgG or the heavy chain constant region of IgG; preferably, the N-terminal and/or C-terminal of the second peptide chain are ligated directly or through a linker to the first protein functional region and/or the second protein functional region;
preferably, the second peptide chain has an amino acid sequence as set forth in SEQ ID NO: 9 or SEQ ID NO: 10.

10. The bispecific antibody according to any one of claims 1 to 9, wherein the first peptide chain and the second peptide chain form a dimer;
preferably, the first peptide chain and the second peptide chain are linked by 2 or 3 pairs of disulfide bonds;
preferably, the first peptide chain and the second peptide chain further comprise a Knob-in-hole mutation;
preferably, the first peptide chain has an amino acid sequence as set forth in SEQ ID NO: 7, and the second peptide chain has an amino acid sequence as set forth in SEQ ID NO: 9 or SEQ ID NO: 10.

11. An isolated nucleic acid molecule, which encodes the bispecific antibody according to any one of claims 1 to 10.

12. A vector, which comprises the isolated nucleic acid molecule according to claim 11.

13. A host cell, which comprises the isolated nucleic acid molecule according to claim 11, or the vector according to claim 12.

14. A method for preparing the bispecific antibody according to any one of claims 1 to 10, comprising steps of culturing the host cell according to claim 13 under suitable conditions, and recovering the bispecific antibody from a cell culture.

15. A conjugate, which comprises a bispecific antibody and a coupling moiety, wherein the bispecific antibody is the bispecific antibody according to any one of claims 1 to 10, and the coupling moiety is a detectable label; preferably, the coupling moiety is a radioactive isotope, a fluorescent substance, a luminescent substance, a colored substance or an enzyme.

16. A kit, which comprises the bispecific antibody according to any one of claims 1 to 10, or the conjugate according to claim 15;
preferably, the kit further comprises a second antibody capable of specifically binding to the bispecific antibody; optionally, the second antibody further comprises a detectable label, such as a radioactive isotope, a fluorescent substance, a luminescent substance, a colored substance or an enzyme.

17. Use of the bispecific antibody according to any one of claims 1 to 10 in the manufacture of a kit for detecting the presence or level of 4-1BB and/or PD-L1 in a sample.

18. A pharmaceutical composition, which comprises the bispecific antibody according to any one of claims 1 to 10 or the conjugate according to claim 15; optionally, the pharmaceutical composition further comprises a pharmaceutically acceptable excipient.

19. Use of the bispecific antibody according to any one of claims 1 to 10 or the conjugate according to claim 15 in the manufacture of a medicament for the prevention and/or treatment of a malignant tumor; preferably, the malignant tumor is selected from a group consisting of rectal cancer, colon cancer, lung cancer, melanoma, liver cancer, gastric cancer, renal cell carcinoma, ovarian cancer, esophageal cancer and head and neck cancer.

20. A method for treating and/or preventing a malignant tumor, comprising a step of administering an effective amount of the bispecific antibody according to any one of claims 1 to 10 or the conjugate according to claim 15 to a subject in need thereof; preferably, the malignant tumor is selected from a group consisting of rectal cancer, colon cancer, lung cancer, melanoma, liver cancer, gastric cancer, renal cell carcinoma, ovarian cancer, esophagus cancer and head and neck cancer.

21. The bispecific antibody according to any one of claims 1 to 10 or the conjugate according to claim 15, which is used for treating and/or preventing a malignant tumor; preferably, the malignant tumor is selected from a group consisting of rectal cancer, colon cancer, lung cancer, melanoma, liver cancer, gastric cancer, renal cell carcinoma, ovarian cancer, esophagus cancer and head and neck cancer.

22. A bispecific antibody, **characterized in that** the bispecific antibody comprises:
(a) an anti-PD-L1 single-domain antibody; and
(b) an anti-4-1BB single-domain antibody.

23. An isolated polynucleotide, **characterized in that** the polynucleotide encodes the bispecific antibody according to claim 22.

24. A vector, **characterized in that** the vector comprises the polynucleotide according to claim 23.

25. A host cell, **characterized in that**, the host cell comprises the vector according to claim 24, or the polynucleotide of claim 23 is integrated into its genome;
or, the host cell expresses the bispecific antibody according to claim 22.

26. A method for producing the bispecific antibody according to claim 22, comprising steps of:
(a) culturing the host cell according to claim 25 under suitable conditions, thereby obtaining a culture containing the bispecific antibody; and
(b) purifying and/or separating the culture obtained in step (a) to obtain the bispecific antibody.

27. An immunoconjugate, **characterized in that**, the immunoconjugate comprises:
(a) the bispecific antibody according to claim 22; and
(b) a coupling moiety selected from a group consisting of detectable label, drug, toxin, cytokine, radionuclide, or enzyme, gold nanoparticle/nanorod, nanomagnetic particle, viral coat protein or VLP, or combination thereof.

28. Use of the bispecific antibody according to claim 22 or the immunoconjugate according to claim 27 in the manufacture of a medicament, a reagent, a detection plate or a kit; wherein the reagent, detection plate or kit is used for: detecting PD-L1 and/or 4-1BB in a sample; wherein, the medicament is used for treating or preventing a tumor expressing PD-L1 (i.e., PD-L1 positive).

29. A pharmaceutical composition, **characterized in that**, the pharmaceutical composition comprises:
(i) the bispecific antibody according to claim 22, or the immunoconjugate according to claim 27; and
(ii) a pharmaceutically acceptable carrier.

30. One or more uses of the bispecific antibody according to claim 22 selected from a group consisting of:
(i) use for detecting a human PD-L1 molecule and/or 4-1BB molecule; (ii) use for flow detection; (iii) use for cell immunofluorescence detection; (iv) use for treating a tumor; (v) use for diagnosis of a tumor; (vi) use for blocking the interaction between PD-1 and PD-L1; and (vii) use for binding to 4-1BB to activate an immune cell.

31. A recombinant protein, **characterized in that**, the recombinant protein comprises: (i) the bispecific antibody according to claim 22; and (ii) an optional tag sequence for helping expression and/or purification.
